# EUROPEAN PATENT APPLICATION

(11) **EP 1 915 997 A1**
(43) Date of publication of application: **30.04.2008**
(21) Application number: 06780703.2
(22) Date of filing: 30.06.2006
(51) Int. Cl.: A61K 36/48, A61K 8/97, A61K 36/18, A61K 36/70, A61P 17/14, A61Q 7/00, A23L 1/30

(54) **AGENT FOR HAIR GROWTH**

(30) Priority: 12.08.2005 JP 2005261312; 01.02.2006 JP 2006024532
(71) Applicant: KOHNO, Kenji, Hadano-shi, Kanagawa 257-0004 (JP)
(72) Inventor: KOHNO, Kenji, Hadano-shi, Kanagawa 257-0004 (JP)
(74) Representative: Rutherford, Jodie
(86) International application number: PCT/JP2006/313146
(87) International publication number: WO 2007/020755

(57) **Abstract**

The aim of this invention is to offer a novel agent for hair growth, which has excellent hair growth effects but not side effects. The agent for hair growth of the present invention is **characterized by** comprising a processed semi-mature soybean and/or a processed semi-mature soybean extracts and at least one substance selected from the group consisting of a processed Polygoni Multiflori Radix, processed Polygoni Multiflori Radix extracts, a processed Cynanchum bungei Decne or processed Cynanchum bungei Decne extracts ,preferably further comprising Longan seed and/or Longan seed extracts as active ingredients. This agent for hair growth has no side effects when used externally or internally, it can notably improve hair growth within a short period of time; ranging from 6 to 12 weeks, can return hair to its normal hair colour (for example from white to black) and can improve the gloss of hair.

## Description

### FIELD OF THE INVENTION

This invention relates to a hair growth. More specifically, this invention relates to a substance which has an excellent hair growth effect and is very safety.

### BACKGROUND ART

Alopecia and thin hair are some of the most common problems for mankind in the world. It is considered that various factors, for example, excessive activation of male hormones, decrease of blood flow to the hair follicle and excessive secretion of sebum etc. may cause alopecia and thin hair. These factors may be cause by aging, stress or/and life styles etc.

Hair restorers, that are being suggested or put on the market today can, for example, consist of various herbal extracts, food extracts and vasodilator chemical compounds, which can eliminate the cause of alopecia and thin hair.

Up to now, it was suggested that isoflavone etc. contents in soybean, had an effects on hair growth, therefore soybean was used as a component of a hair restorer. For example, in the patent document 1 (JP 2001-238637A), it was disclosed that food components which contained extracts of millet and ginkgo leaf and isoflavones extracted from soybean etc. have a positive effect on hair growth. In the patent document 2 (JP 2002-053434 A), it was disclosed that a hair tonic contained isoflavone extracted from soybean and/or kudzu. And, in the patent document 3 (JP H08-127517 A), it was disclosed that black soybean broth was included into an external hair growth tonic and an external anti hair loss agent as a compound. And, it was also suggested to apply herbal medicines, such as Polygoni multiflori Radix etc. to a hair growth tonic.

In the patent document 4 (JP H03-206019 A), it was also described that dark soybean, Polygoni multiflori Radix etc. were used as external components for restoring lost hair. And, in the patent document 5 (JP H06-227946 A), it was described that herbal medicine extracts, such as extracts of ginseng etc. which were extracted with alcohol such as "Huangjiu" (yellow rice or millet wine; a type of Chinese alcohol) etc. were used for promoting hair growth.

Further, in regard to the Sapindaceous plant Longan (Euphoria Longana (Lour.) Steud.), it is common knowledge that components extracted from the flesh of the Longan fruit with water and/or organic solvents had positive effects on hair growth and restoring hair (patent document 6: JP H06-100421 A). And, it was described that The Longan seed extracts have a moisturizing effect and can be mixed into an external skin preventive (patent document 7: JP 2002-145732 A).

However, as mentioned above, the conventional hair tonics (in Japan are classified into the quasi-drug tonics (a term of Japanese Pharmaceutical Affairs Law)), which have components extracted from herbal medicines and foods. These tonics are easily obtainable, but the hair growth effect is insufficient. On the other hand, hair tonics, which are classified as drugs (a term of Japanese Pharmaceutical Affairs Law) and are used as antihypertensive and male hormone inhibitor, have some hair growth effect, but their side effects are severe. Further, in order to be able to purchase drug hair tonics it is necessary to see a doctor and obtain a prescription.

It is therefore understandable, that I am consulted by a lot of patients with hair trouble who claim, that they have never found a useful tonic.
Patent document 1: JP 2001-238637 A
Patent document 2: JP 2002-053434 A
Patent document 3: JP H08-127517 A
Patent document 4: JP H03-206019 A
Patent document 5: JP H06-227946 A'
Patent document 6: JP H06-100421 A
Patent document 7: JP 2002-145732 A

### DISCLOSURE OF THE INVENTION

### Problem to be solved by the invention

The aim of this invention is to offer a novel agent for hair growth, which has excellent hair growth effects but not side effects.

### Means for solving the problem

The inventor of this invention found that the processed semi-mature soybean has significantly enhanced effects on hair growth compared to the raw or fully matured form. The processed semi-mature soybean was obtained by removing the typical taste of the raw soybeans without pulverizing them by excessive heating. On completion of this invention, the inventor established that the processed semi-mature soybean plays an important role as a ingredient in the agent for hair growth.

In the agent for hair growth of the invention, the inventor combined the processed semi-mature soybean and the processed Polygoni Multiflori Radix or the processed Cynanchum bungei Decne to increase synergy effect on hair growth. In here, the processed Polygoni Multiflori Radix and processed Cynanchum bungei Decne were made from raw Polygoni Multiflori Radix and raw Cynanchum bungei Decne, obtained by soaking them in dark soybean extracts and alcohol. Further, the color and gloss of hair can be improved by increasing the content of Longan seed. In addition, in the agent for hair growth of the invention, the extracts of the processed semi-mature soybean, the processed Polygoni Multiflori Radix, the processed Cynanchum bungei Decne and the Longan seed can be combined.

The agent for hair growth of the invention can be taken orally or can be added to foods or drinks. On the other hand, it also can be prepared as an external product.

### Effect of the invention

In this invention the positive results of the agent for hair growth is based on the use processed semi-mature soybean, processed Polygoni Multiflori Radix, processed Cynanchum bungei Decne, and Longan seed etc. as active ingredients. Especially, this agent for hair growth has no side effects when used externally or internally, it can notably improve hair growth within a short period of time; ranging from 6 to 12 weeks, can return hair to its normal hair colour (for example from white to black) and can improve the gloss of hair.

Moreover, existing hair tonics usually are offered as capsules or tablets types and therefore not convenient to take, because they resemble drugs/medication. The agent for hair growth of the invention has not only an excellent hair growth effect, but also can be regarded as a delicious food, for example in form of a cookie, a.rice cracker, an Okaki (a Japanese cake), an Arare (a Japanese cake), Japanese cakes and cracker etc..., which might, in the opinion of the inventor enhance the use. In this sense, the agent for hair growth of the invention is an important chapter in the hair tonic history.

### BRIEF DESCRIPTION OF THE DRAWING

Figure 1 shows changes in the degree of alopecia and thin hair before and after taking the agents of hair growth of this invention. There are significant results of hair growth in the following groups; the compound ingredients 1 group, the compound ingredients 2 group and the compound ingredients 3 group.
Figure 2 shows a subject' s hair condition before and after taking the agent of hair growth (A: Before taking, B: after taking). These data support the results shown in figure 1. The hair growth effect of the compound ingredients 1 was confirmed in this figure.
Figure 3 shows a subject's hair condition before and after taking the agent of hair growth (C: Before taking, D: after taking). These data support the results in figure 1. The hair growth effect of the compound ingredients 2 was confirmed in this figure.
Figure 4 shows a subject's hair condition before and after taking the agent of hair growth (E: Before taking, F: after taking). These data support the results figure 1. The hair growth effect of the compound ingredients 3 was confirmed in this figure.
Figure 5 shows changes in the pain pattern at the hair root before and after taking the agent of hair growth in this invention. The sensation of pain was increased in the groups of the compound ingredients 1, the compound ingredients 2 and the compound ingredients 3.

### THE BEST MODE OF THE INVENTION

What is hair growth? "Keeping the current hair health, preventing hair loss and increasing the strength of hair", is the definition by the Japan Hair Science Association. The effects of hair growth in this invention basically support this definition. To be more specific, it can improve hair thickness, hair color and hair gloss. New hair grew on the scalp where hair had already been lost, in other words, it enhances hair-growth (in Japanese pronunciation is "Hatsumou") . The meaning of the hair growth effects in this invention includes this "Hatsumou" effect, the meaning has a wide sense.

Now, I want to explain the manufacturing methods of each component within the invention such as the processed semi-mature soybean, the processed Polygoni Multiflori Radix, the processed Cynanchum bungei Decne and Longan seed and the composition ratio of the invention. I also want to explain how to use the invention as follows.

### The components in the invention

### "The processed semi-mature soybean"

The raw material of the processed semi-mature soybean in this invention is soybean (Binomial name; Glycine max (L.) Merr.), it belongs to Glycine, Fabaceae and the color of its seeds is yellow. This kind of soybean originates in China. It was brought to Japan during the Yayoi period and was permeated during the Kamakura period.

In Japan, soybeans are mainly classified as yellow soybeans, blue soybeans, black soybeans according to the color of the seed coat. Yellow soybeans are usually called "soybean (Daizu)", and are generally used as raw material for boiled soybean, natto, bean curd and miso etc. Blue soybeans are used as rawmaterial for soybean flour and Japanese-style confectionery. Black soybeans are usually called "black soybean (Kuromame)" and are used as food.

Any kind of soybean can be used as raw material for the processed semi-mature soybean in this invention. The yellow soybean however is especially suitable for the processed semi-mature soybean. Further, soybeans are subdivided into large-grained, kmiddle-grainedand small-grained soybeans, according to the size of the seeds. Any sizes of soybean can be used for this invention. Furthermore, any soybean strain can be used for this invention, too.

For this invention soybeans with its seed coat or seed bud removed, or soybeans with its seed coat and seed bud removed can be used as well as soybeans irradiated with gamma rays.

It was reported that sitosterols, lipids, peptides, isoflavones, saponins and polysaccharides extracted from soybeans or soybean ferment extract etc. have hair growth effect. However, raw soybean has a stimulating effect on the stomach and according to JP 2004-182687 A, reactants of the enzyme hydrolysis of soybean proteins have a "hair growth inhibitor effect" instead of a hair growth effect. For this invention, raw soybeans were processed to the processed semi-mature soybeans. These processed semi-mature soybeans are save, have high hair growth effects and thus can be used for the invention.

In this invention, "the processed semi-mature soybean" refers to soybeans that were prepared with heat in order to remove their typical taste found in their raw form without pulverizing them by excessive heating.

For the heating process any kind of heater can be use for this invention, for example, gas stove, oven, microwave oven, toaster etc. In laboratories and factories hot plates, a mantle heater, an electric furnace, a mule heater, a Maki bill type heater, a throw type heater, a lab heater, an electric stove and etc. Moreover, any small cooking tool can be used.

The methods for making processed semi-mature soybeans are not restricted to the following methods. Moreover, heating conditions may vary due to the following factors; modality of the heat instrument, volume of the soybeans and whether the soybean was soaked in water or not, etc.
(1) Preheat a frying-pan on a gas stove, then put the soybeans into the frying-pan, stir the soybeans from thirty seconds up to ten minutes (desirably one to five minutes) while keeping the temperature a the same, medium level.
(2) Put the soybeans in boiling water (90°C to 100°C) for three seconds up to sixty minutes (preferably ten seconds to ten minutes, more preferably thirty seconds to three minutes) to heat the beans. Instead of the hot water, any kind of food oil (for examples, salad oil, sesame oil, frying oil, safflower oil, olive oil and processed element oil, and its kinds are not restricted especially) can be used. The oil temperature was between 60°C to 480°C, prefrerably 100°C to 300°C, more preferably 180°C to 250°C. Other than boiling water and food oil all edible liquids can be used.
(3) Soak soybeans in warm water (within the range of 30°C to 90°C, preferably 60°C to 80°C) for ten minutes to three days.
(4) Put the soybeans into a steamer etc. and heat the beans by using steam of 100°C to 380°C (preferably 150°C to 300°C) for three seconds up to sixty minutes (preferably ten seconds to ten minutes, more preferably thirty seconds to three minutes.)
(5) Heat soybeans by using an oven at the range of 200°C to 220°C for 20 minutes, then at 170°C for 30 minutes, and then at 150°C for 40 minutes.

### "Processed Polygoni Multiflori Radix"

The processed Polygoni Multiflori Radix in the agent for hair growth of this invention was obtained by soaking black soybean extracts and liquors to raw Polygoni Multiflori Radix. Polygoni Multiflori Radix (Heshouwu) is the dry root of Polygonum Multiflorum (binomial name: Polygonum multiflorum Thunb.). It was recorded in the book "Kai Yuan Ben Cao" during the Song Dynasty in China. The dry root is referred to as "raw Polygoni Multiflori Radix", and that treated with heat as "mature Polygoni Multiflori Radix".

Polygoni Multiflori Radix includes components of various kinds of dianthrones such as emodin, rhein, aloe-emodin, chrysophanol, physcion, etc. as well as components of 2, 3, 5, 4-tetrahydroxystilbene-2-O-beta-D-glucoside, 3-methyl-1, 6, 8-tri-hydroxyanthraquinone, 2,6-dihydroxy-benzoic acid, 1,2-propanediol-1-(4-hydroxy-phenyl), emodin-8-O-beta-D-glucoside, 8-O-beta-D-glucopyranoside, chrysophanol 8-O-beta-D-glucopyranoside, torachrysone 8-O-beta-D-glucopyranoside, aloe-emodin 8-O-beta-D-glucopyranoside, chrysophanol 8-O-beta-D-(6'-O-malonyl)glucopyranoside, (+)-lyoniresinol-3-alpha-O-beta-D- glucopyranoside, 2,3,4',5-tetrahydroxy-trans-stilberie-2-O-beta-D-glucopyranosi d,2,3,4',5-tetrahydroxy-trans-stilbene-2,3-di-O-beta-D-glucop yranoside, Me indole-3-(L-alpha-amino-alpha-hydroxypropionate), citreorosein, Me gallate, anthraquinones, sennoside A, sennoside B, etc.

Further characteristics of Polygoni Multiflori Radix include containing a large amount of Ca, Fe, Cu, K, Mn, Zn, and P for the average content of the plant.

Polygoni Multiflori Radix contains a kind of Antorakinon component. Therefore, if taking Polygoni Multiflori Radix in its raw form, an individual will either develop loose stool, light diarrhoea or diarrhoea. For this reason Polygoni Multiflori Radix is usually taken in its mature form. Mature Polygoni Multiflori Radix has the effects of nourishing and strengthening and is used to treat tinnitus, grayhair, sleeplessness, defective sperm growth, decline of the lower body, hyperlipemia, chronic hepatitis, neurasthenia, etc. Recently, based on its effect of decreasing serum cholesterol, it has been added to various hair tonics to control the sebum secretion of the skin on the scalp (for example, JP H02-48514 A, etc.). However, when used on its own mature Polygoni Multiflori Radix is insufficient in stimulating hair growth, and so is often used in combination with other medicinals.

On the other hand, black soybean belongs to Glycine, Fabaceae as previously stated, and the colour of the coating of its seed is black. Within Japan, Hokkaido and Okayama Hyogo Prefecture are its chief districts of production. It is thought to be effective in the treatment of thrombosis, hypertension, liver complaints, diabetes, common stone diseases, arthritis, weight loss, and cancer prevention. Black soybean is prepared in a variety of ways, including as a tea, milk, bean cake, cake, Okaki, miso, and stew.

Moreover, liquors that are in general use such as those used for drinking or cooking can be used to process the processed Polygoni Multiflori Radix. The liquor "Huangjiu" is especially suitable in stimulating hair growth. Huangjiu is an alcohol that ripens for a fair amount of time (from 1 year to several decades), and its raw material is chiefly glutinous rice and wheat malt. It's name huang jiu - Chinese = 'yellow wine' - is derived from its yellow colour, with a brown or amber glow. Since it is ripened for an extended period of time, it is also known as lao jiumeaning "aged liquor" in Chinese. It was introduced to Japan by Nakagawa Tadahide in the Edo period (Nakagawa Tadahide: "SinZokuKiMon", year 1799 A. D.). "Shao xing jiu" is one kind of Huangjiu well-known in Japan. The alcohol content of Huangjiu is roughly 14-20%. Depending on the time it is given to ripen, there are a fewdifferences in the percentages. Huangj iu is commonly used for cooking in China, its country of origin.

For the purpose of deriving a sufficient hair growth-stimulating effect of the Polygoni Multiflori Radix used in this invention, I soaked the Polygoni Multiflori Radix in black soybean extract and liquor (Huangjiu), to then use the resulting product as the "processed Polygoni Multiflori Radix". In this case, the above-mentioned "Black soybean extracts" are hot-water extracts which are obtained by boiling black soybeans.

For this invention, the processed Polygoni Multiflori Radix was obtained by drying the compound ingredients which were then soaked together with the black soybean extract and the liquor. It is desirable that these processes were condensed with a cooker whilst heating. There are other processing methods one can use for obtaining processed Polygoni Multiflori Radix, therefore, one is not restricted to the method mentioned above.

### "Processed Cynanchum bungei Decne"

For this invention, the processed Cynanchum bungei Decne was used as the raw material to obtain the processed Cynanchum bungei Decne , which is the name of an herbal medicine (brand name). Usually, Cynanchum bungei Decne. (binomial name) is readily available in herbal medicine markets. However, Cynanchum auriculatum Royle (Asclepiadaceae), Asclepias hastata Bunge. Enum., Cynanchum hastatum Lamarck, Symphyoglossum hastatum (Bunge) Turczaninow, Vincetoxicum hastatum (Bunge) Kuntze are all marketed as raw Cynanchum bungei Decne though is a small amount, too. Inotherwords, the compounds contained within Cynanchumbungei Decne occur in several kinds of plants. This is to be admitted in China.

The chief area of production of Cynanchum bungei Decne is Shandong province, China. Its natural growth in Japan has not been confirmed.

The compounds contained within Cynanchum bungei Decne include Bungeiside A, Bungeiside B, Bungeiside C, Bungeiside D, Blumenol, (-)Leucanthemitol, beta-Sitosterol glucoside, 4-Hydroxyacetophenone, Cynanchol, etc.

All the parts of Cynanchum bungei Decne . have pharmacologic effects. Usually, the parts used include the root, stalk, and wisteria. The dried parts are referred to "raw Cynanchum bungei Decne". It is used as an antiasthenic drug due to its effect of improving the function of the liver, kidneys and blood.

For this invention, the processed Cynanchum bungei Decne was obtained by using the same method that was employed in the processing of the Polygoni Multiflori Radix, i.e. soaking the raw Cynanchum bungei Decne in black soybean extract and liquor (Huangjiu) and heating, then drying it to obtain the processed Cynanchum bungei Decne.

### "Longan seed"

For this invention, "Longan seed" is the seed of the Longan plant (Binomial name: Euphoria Longana (Lour.) Steud.). It is the fruit of an evergreen tree and belongs to the Sapindaceae family, growing from South Asia to Southeast Asia and China. In China, the flower of Longan usually blooms from March until April, and its fruits are ripe from July until October. Longan contains components such as Uridine, 5-(Hydroxymethyl)-2-furfuraldehyde, 1,1-dimethyl-2-propenyl 1-O-β-D-glucopyranoside, Et beta-D-glucopyranoside, 5-(hydroxymethyl)-2-furfuraldehyde, alpha-and beta-amyrin, lupeol, 24-methylenelanost-8-en-3beta-ol, 24-methyleneparkeol, 24-methylenecycloartanol, 4 4-methyLongan seedteroLongan seed, 64-desmethyLongan seedteroLongan seed, etc.

Longan fruit is composed of the husk (testa part) , the flesh (provisory testa) and the nucleus (seed). Among these parts, the meat of has a peculiar sweetness with thin smell and is consumed as a food. In China, the dried meat is known as "Longan meat" and is recognized as having a "blood tonifying" effect. In other words, it improves the circulation of the blood as well as its function and therefore has been used since antiquity within the herbal medicine pharmacopeia. Moreover, in Japan the blood-tonifying effect of Longan meat is recognized, contributing to the Japanese Pharmacopeia as a traditional herbal medicinal, and is used as an insurance drug.

Until today, Longan seed is considered worthless as a food or medicinal substance and therefore its value abandoned.

As far as the application of the hair growthtonic is concerned, Longan seed can be used for either 'damp' or 'dry' conditions. It is thought that the hair growth effect is further increased when Longan seed is processed in a similar way to the Polygoni Multiflori Radix.

### "Extract of each ingredient"

Extracts obtained from processed semi-mature soybean, Polygoni Multiflori Radix, Cynanchum bungei Decne and Longan seed with solvents, as well as dried powders of these extracts, can be used for the agent for hair growth. The extracts obtained from a prepared mixture comprising the powders of the processed semi-mature soybean, Polygoni Multiflori Radix, Longan seed and Cynanchum bungei Decne can be used. In addition, it is possible to use a combination of non-extract and extract substances.

The above extracts can be used for the agent for hair growth after one of the following procedures have been completed: concentrating the above extracts, dissolving the dry extracts into water or a polarized solvent, or refining (such as through bleaching, deodorization, desalination, etc.), or fragmenting those extracts that are within the range in which their functionality is not compromised, with the use of a chromatograph.

The above mentioned unprocessed as well as processed (fragmented) extracts may be frozen or dried after being processed (or fragmented). The resulting matter can be dissolved into a solvent or can be wrapped to beshicl and the micro capsule etc. such as riposorm prior to use.

Next, I would like to explain the methods of processing for the above mentioned extracts.

When preparing extracts of each active ingredient contained within the agent for hair growth, it is desirable that processing is done after either cutting, drying, or crushing each of the raw materials to enhance extraction efficiency,' it is possible to extract the active ingredients by using a supercritical fluid and a sub-critical fluid, or by soaking in a solvent. To increase the extraction efficiency when using the extraction solvent, it is better to stir the solvents or to homogenize the extraction in the solvents. Regarding the temperature for extraction, it is desirable that it be within a range of around 5°C to 10 degrees lower than the boiling point of the solvent used. As far as the extraction time is concerned, although it differs depending on the kind of solvents along with the temperature used for extraction, it is desirable to be from about 1 minute to roughly 14 days.

One is not especially restricted in regard to the kind of extraction solvents used. Water and the org-solvents are enumerated, for instance, the following materials may be used as an org-solvent : alcohols and butanols such as methanol, ethanol, 1-propanol, 2-propanol, n-butanol, s-butanol, t-butanol, ethyleneglycol, 1,3-butyleneglycol, propyleneglycol, dipropyleneglycol and glycerin etc.; ethers such as diethyl ether and dipropyl ether; esters such as butyl acetate and ethyl acetate etc.; ketones such as acetone and ethyl methyl ketone etc.; organic acids such as formic acid and acetic acid etc. , dimethyl sulfoxide, acetonitrile, tetrahydrofran, dioxane, and phenols, and other parents oily org-solvents (such as chloroform, pentane, hexane, cyclohexane, benzene, toluene, xylene and vegetable lipids such as colza oil). It is possible to use these solvents by selecting one or several among these. From a safety standpoint, water or ethanol is desirable. Moreover, physiologicalsaline, phosphoric acid buffer and phosphate buffer saline etc. may be used. In addition, one may also select a supercritical fluid and a sub-critical fluid such as water, carbon dioxide, ethylene, propylene, ethanol, methanol, and ammonia, etc.

### "Other ingredients"

Aside from the active ingredients mentioned above, the agent for hair growth in this invention can also be used by combining foods and beverages, raw food substances or medicines, etc. For example, mixing raw materials that improve health, natural medicinal materials (such as curative plants, or curative animal products or minerals), traditional Japanese drugs, traditional Chinese drugs, Westernmedicines orother compounds with the extract material. Some exmples are enumerated below.

Tacasabrou is enumerated as one of the natural medicinal materials. Tacasabrou (Eclipta prostrata L.) belongs to the Asteraceae family. Its Japanese name is Tacasabrou, is known as Hanliancao, Mohanlian, Lichang or Lichangcao in Chinese and is referred to Eclipta in English. It has the effect of blackening hair (which is enhanced when used together with ginger). Tacasabrou is an annual herb widely distributed from the temperate zones to the tropics. In Japan, it grows in Honshu and the southern regions, however not in Hokkaido. It grows in rice fields, in proximity to levees, and waterway walls, etc. and is also referred to as a rice field weed. It has white flowers that blossom from summer to autumn and seems to have become a naturalized plant originally brought from China with the introduction of rice farming. After World War II, America introduced Tacasabrou to Japan and became a naturalized plant.

For this invention, the Tacasabrou from any region may be used. Tacasabrou has an antibacterial, anti-viral and anti-inflammatory effect and has been used as a curative plant in China for 1000 years or more. From a traditional Chinese medicine perspective, Tacasabrou is used to tonify the Kidneys (referred to in Japanese as "HoJin") as well as treat various hemorrhagic conditions.

As other natural plants or animals or minerals though it is not restricted especially, for instance, the following can be enumerated; Mutongzi, Akebi's Manxingjing, Mutong Ashitaba, Asunaro leaf, Gancha, Ganchaduru, Amadokoro, Yamaren, Araragi, Awabirui's gara, Shijueming, Ikarikusa, Yuliren, Izui, Yiwei, Itijiku's mi, Itijiku's leaf, Ityou, Dao miao, weilingxian, Yanqianshe, Yanlu, Yinchenhao, Mianyinchen, Yinyanghuo, Uikyou, Ukonn, wutou, wuji, wuzheigu, wumei, wuyao, Utubogusa's huashui, Tuyuliang, Guobaijian, Uwaurusi, eijitu, ezoukogi, yanhusuo, yan ming cao, huangqi, ougonn, jianhuang, huangjing, huangtu, huangbo, yingpi, wangbuliuxing, huanglian, ootudirahuji, fangji, oobako'szhongzi, cheqianzi, oobako's quancao, cheqiancao diqiecao, yuanzhi, xiagucao, kaika, haijinsha, huailiuqi, gositu, liuqi, jiezi, haisongzi, haitongpi, hairencao, makuri, jiubai, aiye, yomogi, kaoosi, kaki's leaf, kaki's heta, xiagucao, kezi, Gajyutu, Kakkou, Gegen, huashi, kanokonnu, kamiture, kayaturikusa, kayanomi, garana, Kariraku, kezi, hualinomi, mugua,lougen, karokonn, kalousji, kalouren, chuanliu, kawaratake, jixingzhu, kawarayomogi, yinchehao, ganjiang, guanzong, guanzhong, gancao, dongbeigancao, xibeigancao, kannzou, zhigancao, guanzhong, kuandonghua, kuandonggen, Hanliancao, kikyou, jigen, juhua, hangjuhua, zhizi, zhishi, zhike, kisasage, jixing, jixingzhu, beishashen, jicaogen, jipi, kihada, huangbo, guiban, gimunemasirubesuta, huguama, qianghuo, xingren, yuzhu, kirannkusa, jinyingzi, jingan, jinyinhua, yinchaihu, jinzhencai, jinqiancao, Mizuhik, Xianhecao, fruit of Guaba, Panguo, Guaba, Goujizi, kukonomi. Kukonogenpi, digupi, goujiye, kushen, kuzunonemodo, gegen, gouji, kutinasi, Juma, Kumassa, Xiongliu, kurumi, kuchenpi, heiwenzi, sangca, sangye, sangdeshi, keigai, jingjie, jixueteng, guizhi, guipi, guitongguipi, guangnanguipi, dongxingguipi, yueguishiye, ketumeixi, maorenshen, qianniuzi, kennjitu, quanshen, xuanshen, gennnosyouko, miaoyi, maiyatanghuayi, honghua, hehuanpi, hangjuhua, jiangxiang, kousi, xiangxu, hongshen, xiangfuzi, genmi, houpi, houpishi, haoben, niuhuang, wujiashen, niuxi, huainiuxi, cuanxi, wuzhuyu, gushuibu, hutaorou, wubeizi, hupo, niupangzi, huma, wuweizi, huyuba, caihu, caihumu, xixin, zakuroguopi, shiliuguopi, sahurann, shuoyang, shanguilai, shanzhazi, shanzharou, shancigu, shanjizi, chansha, Shanzhuyu, shanjiao, shandougen, Shaunzhaoren, shanbiandou, shanyao, Sanlin, dihuang, shudihuang, ziyan, zihuadiding, sigualou, chiwujia, digupi, zigen, yingzigen, ruanzigen, chishi, zishiyan, zishuzi, lizi, Jilizhi, siturisi, Difuzhi, Jiugancao, chishizhi, shaoyao, daheshaoyao, daoxieshaoyao, chishao, shechuangzi, shashen, beishashen, cheqianzi, cheqiancao, syamusi, chongweizi, xiuzhifuzi, shudihuang, shusha, zonglushi, shengjiao, syouga, songzi, xiaomai, cangpu, cangpugen, shengma, jiaomu, xiaolianqiao, diqiecao, nuzenzi, dilong, dilonggan, xinyi, Jinqi, shenju, liushenju, qinjiu, suikazuradejingye, rendong, suikazuranohana, jinyinhua, shuizhi, sugina, dougu, xianggu, seisousi, qingdai, qingpi, xiyangrenshen, chishao, shicanggen, shisongzi, shiliuguopi, shijieming, shigao, shidou, jiegupu, zeratinn, xianhecao, quanxie, cuanxiong, cuanyujin, qianhu, cuanke, cuangu, cuanxi, xicaogen, cantui, sennna, cuanbei, xuanfuhua, sennburi, xianmao, Chuanglianzi, caoguo, zhaojiaochi, zhaojiaozi, Zhaojia, Zhuyazhaojia, shangshenzi, shangdeshi, cangerzi, cangshu, gulicangshu, caodou, samgbaipi,sangqiao, sangye, sangdeye, chebuye, shumu, shuye, dahuixiang, dahuang, jinwendahuang, dahaizi, dagencao, dagendezhongzi, luopuzi, daizheshi, daqingye, Dazhao, nazime, dafupi, gaoyuancao, zhexie, zhelan, Ta-merikku, yujin, kuragenpu, danshen, danzhuye, zhuye, tannpopodegen, pugongyinggen, zhuru, Zhuru, zhujierenshen, zhuye, zhimu, chaye, diyu, dixiang, dizi, diaotenggou, zhuling, chenpi, tsuchiakebi, tutongchao, luchao,Tsurudokudami's root, Tsuruna, bingqianshe,Tinglizhi, Tioanzhuhuang, tianzhuhuang, tiannanxing, tianma, tianmushi, tianmuman, Tianmu, tianmendong, Donggua, danggui, dahedanggui, dongkuizi, tnaghuma, dengxin, dengxinchao, dangshen, daodou, dongchongxiachao, tangduhuo, tao ren, chenpi, dangyao,Senburi, tuguagen, tuguagen, tuguashi, duhuo, tangduhuo, yuxingchao, Dokudami, Jyuyaku, tusizi,Tochi fruits, duzhong, tutongchao, tufulin, shanguilai, tubiejia, biejia, Liaobuzhi (Toritomarazu), mumu,Tonburi, Difuzhi, Nazuna, Natamame, baidaodou, roudou, roudoukou, Natsumeru, Narukoyuri roots, huangjing, nantianshi, nantianye, nanmanmao, Nigaki, Rouchongrong, roudou, jinmu, ruxiang, Niwatoko, Niwatoko leaf, Ninjin, Gosyuninjin, shengganrenshen, shensairenshen, baishen, qushen, maorenshen, rendong, Nezu fruits, dusongzi, Nezumimochi, nvzhenzi, beimu, chuanbeimu, chuanbei, maiya, baijiezi, boziren, baidou, baixianpi, baitouweng, baitaohua, baidaodou, bainantian, nantianshi, baibiandou, biandou, manmendong, kaibianmaimendong, bagetian,pohuzhi,Hakobe,biajiaoshi,Lianzhi,bajiao,dahuixiang, bohe, Juemingzhi, Paozhi-Juemingzhi, Hanamotsu, Habu-kusa, Houji-Habucya, binqianshe, binfangfeng, panguo, banxia, Banzakuro leaf, banzhilian, panshiliuye, pangdahai, dahaizi, bannagen, Hikai, bianhua, bianhuadege, Hikiokosi, yanmingchao, maorenshen, maorenshen, lindeshi, ganye, mazi, tanghuma, baihe, baizhi, baishu, baitan, baiwen, baibugen, baihuasheshechao, Baijiangchan, baijiangchan, hiru, shuizhi, pipaye, binglangzi, fupenzi, fuling, fuzi, paofuzi, Biejia, tubiejia, Hongyinghua, bianxu, fangyi, maogen, mangchong, fangfei, puhuang, Bokusou, pugongyinggen, buguzhi, pohuzhi, mudanpi, muli, kuihua, mahuang, qujiemahuang, mahuanggen, Makuri, hairenchao, maziren, Matatabi, tianmuman, Matatabi fruits, tianmushi, chongliu, songdeshi, haisongzi,'songye, songteng, Matsuyani, songzhi, manjinzi, mimenghua, Mitsurou, Sarasimituryou, Mirobaran, kezi, wuhuaguo, wuhuanguo, wuhuaguoye, wuhuanguo leaf, mumu, Mugunoki, mianyinchen, muzhe, mutong, mutianmiao, Matataminoryu, mugua, muxiang, toadeye, menjin, shegan, yizhi, yizhiren, yimuchao, yejiaoteng, shicheshi, Yatsumeran, xiaongdan, Yukinosita, Yuzuri leaf, Baihe root, baihe, Yuyiren, Aiye, aiye, leiwan, laizhi, luopuzi, luohanguo, luopuzi, ligenpi, longyanrou, liujinu, longgu, Magnesium sulfate, longdan, liangjiang, gaoliangjiang, lvdou, lingzhi, lianqiao, lianqianchao, Kakitoosi, lianrou, Lianzhi, lurong, lufengfang, lulutong, heqianghuo, honghua, liulonghuang, lurongdabiwan, zhiyungao, Herbal medicine plaster of Utida, Bath preparation of Utida, yunnanhuangjin, yunnanpianyujin, yunnantianqi, tianqirenshen, lingzhi, lingzhifeng, New Caihor ionizing calcium of Ki chitosan and Tida of agaricus processed food and Utida, Asreb, fengmi, and adlay processed food, Pual tea, Caki No leaf, Nacacha, and Megsrinoki tea, etc. Moreover, the following herbal medicines can be enumerated, too. Prunella vulgaris L., Prunella hispida Benth., Ophioglossum pedunculosum Desv., Ophioglossum thermale Kom. , Ophioglossum reticulatun L., O. petiolatum Hook., Cirrhopetalum henryi Rolfe, Stephania graciliflora Yamamoto, Securinega suffruticosa (Pall.) Rehad., Lepisorus contortus (Christ) Ching, Senecio dianthusFranch., Erigeron annuus (L.) Pers., Tetrastigma delavayi Gagn., Veronica spuria L., Achillea alpina L. Achillea wilsoniana (Heim.) Heim., Indigofera pseudotinctoria Matsun., Indigofera parkesiiCraib, Anotis ingrata (wall.) Hook. f., Begonia wilsonii Gagn., Ligularia nelumbifolia (Bur. et Franch. )Hand. -Mazz., Parochetus communis Ham., Kyllinga monocephala Rotth., Pittosporum glabratun Lindl., Solidago virga-aurea L. var. leiocarpa (Benth.) A. Gray, Indigofera pseudotinctoria Matsum., Maianthemum bifolium F. w. Schm., Schisandra bicolor Cheng, Limonium bicolor (Bge.) O. Ktze., Piper boehmeriaefolium wall. var. tomkinense C. DC., Rosamultiflora (Thunb.) var. platyphylla Thory, Mahonia bealer (Fort.) Carr., Mahonia fortunei (Lindl.) Fedde Mahonia japonica (Thunb.) DC., Syzygium cromaticum (L.) Merr. Et perry, Erycibe obtusifolia Benth., Syzygium aromaticum (L.) Merr. et Perry, Zornia diphylla Pers., Cornus macrophylla wall., Syzygium aromaticum (L.) Merr. Et Perry, Zornia diphylla Pers., Gynostemma pentaphyllum (Thunb.) Mak., Schefflera arboricola Hayata, Schefflera venulosa (wight et Arn.) Harms, Buddleia lindleyana Fort., Macropanax rosthornii (Harms) C.Y. wu ex Hoo, Mosla cavaleriei Levl., Actinodaphne obovata Bl., Alocasia cucullata (Lour.) Schott., Akebia quinata (Thunb.) Decne. , Akebia trifoliate (Thunb.) Koidz., Akebia trifoliate (Thunb.)Koidz. var. australis (Diels) Rehd. Holboellia latifolia wall., H.angustifolia wall., Lampetra japonica (Martens), Hydrangea macrophylla (Thunb.) Ser. , GaliumaparineL. Galiumasperifolium wall., Cacalia ainsliaeflora (Franch.) Hand.-Mazz., Dysosma pleiantha (Hance) woods. , Dysosma veitchii (Hemsl. et wils.) Fu, Alangium chinense (Lour.) Harms, Alangium platanifolium , Harms Alangiumlamarckii, Illicium verum Hook. f., Illiciumlanceolatum A. C. smith, Panax ginseng C. A. Mey., Herminium monorchis (L.) R. Br., Panax ginseny C. A. Mey, Dracontomelon dao (Blanco) Merr. et. Rolf, Dracontomelon dao (Blanco) Merr. et. Rolf, Zanthoxylum nitidum (Roxb.) DC., Helminthostachys zeylanica (L.) Hook., Euphorbia hylonoma Hand.-Mazz., Cayratia corniculata (Benth.) Gagn., Sarcandra glabra (Thunb.) Nakai, Bauhinia championi (Benth.) Benth., Thesium longifolium Turcz., Thesium himalense Royke, Silene tenuis willd., Silene tenuis willd. var. rubescens Frahch., Murraya paniculata(L.) Jack., Polygonum rude Meissn., Aspongopus chinensis Dallas, Asplenium varians wall., Anemone altaica Fisch., Cyperus alternifolius L. subsp. flabelliformis (Rottb.Kukenth.), Bauhinia championi (Benth.) Benth., Murraya paniculata(L.) Jack., Calanthe discolor Lindl., Calanthe tricarinata Lindl., Euphorbia hylonoma Hand.-Mazz., Peristrophe japonica (Thunb.) Brem., Canavaliagladiata (Jacq.) DC., Ainsliaea triflora (Buch.-Ham.), Canavalia gladiata (Jacq.) DC., wikstroemia indica (L.) C. A. Mey., Panax pseudo-ginseng wall. var. notoginseny (Burkill) Hoo&Tseng, Panax pseudo-ginseng wall. var. elegantior (Burkill) Hoo&Tseng, Panax pseudo-ginseng wall. var. wangianus (Sun) Hoo&Tseng, Panax pseudo-ginseng wall. var. bipinnatifidus (Seem.) Li, Sparganium stoloniferum Buch.-Ham, Sparganium simplex Huds., Sparganium stoloniferum Maxim., Gynura segetum (Lour.) Merr., Campylotropis cavaleriei (Levl.) C. Y. wu, Ribes tenue Jancz. Var. viridi. Florum Cheng, Scopolia acutangula C. Y. wu et C. Chen, Saururus chinensis (Lour.) Baill., Viola tricolor L., Oxalis griffithii Edgew. Et Hook. f., Parthenocissus heterophylla (B1.) Merr., Chlorophytum laxum R. Br., Pachysandra stylosa Dunn, Cinnamomum tamala (Ham.) Nees et Eberm. , Lysimachia insignis Hemsl., Anaphalis bicolor (Franch.) Diels, Neocinnamomum delavayi H. Liou, Cimicifuga acerima(Sieb. Et Zucc.) Tanaka., Lindera obtusiloba Bl., Vicia unijuga A. Br., Trifolium repens L., Cyperus iria L., Berberis sargentiana Schneid., Berberis brachypoda Maxim., Berberis dictyophylla Franch. Var. epruinosa Schneid., Berberisdiaphana Maxim.,Tylophora ovata(Lindl.)Hook. et Steud., Tylophora crebriflora, Evodia tepta (Spr. ) Merr. , Evodia lepta (Spr.) Merr., Saururus chinensis (Lour.) Baill., Clerodendron serratum (L.) Spr., Clerodendron serratum (L.) Spr. Var. amplexifolium Moldenke, Potentilla freyniana Bornm., Potentilla freyniana Bornm. Var. sinica Migo, Scleria elata Thw., Potentilla freyniana Bornm., Enteromorpha clathrata (Roth)Grev., Enteromorpha prolifera (Mull) J. Ag., E. cinoressa(L.) Grev., E. intestinalis (L.) Link., Zingiber officinale Rose., Rhus verniciflua Stokes, Brassica chinensis L., Rehmannia glutinosa (Gaertn.) Libosch., Rehmannia glutinosa Libosch., F. hueichingensis (Chao et Schih) Hsiao, Corydalis tomentella Franch., Ipomoea hungaiensis Lingelsh. Et Borza., Odontobutis obscura (T. et S.), Discolia vittifrons Sch., Evolvulus alsinoides L. , Illicium henryi Diels, Illicium yunnanensis Franch., Talinum paniculatum (Jacq.) Gaertn., Senecio Chrysanthemoides DC., Rumex madaio Mak., SInula helenium L., Equisetum debile Roxb., Roscoea intermedia Gagn., Bolbostemma paniculatum (Maxim.) Franquet, Achyranthes bidentata B1., Acyranthes longifolia Mak., Achyranthes aspera L., Stanlianthus thorelii gagn., Platanthera chlorantha Cust. Ex Rehb., Melothria indica Lour., Melothria heterophylla (Lour.) Cogn., Arisaema intermedium B1., Asparagus filicinus Ham. ex D. Don, Asparagus meioclados Levl., Aralia cordata Thunb., Angelica gigas Nakai, Lysimachia insignis Hemsl., Hypericum bellum L., Hymenodictyon excelsum (Roxb.) wall., Unio sp., Hedychium coronarium Koen., Hedychium spicatum Ham., Clerodendron trichotomum Thunb., Odontobutis obscura (T. et S.), Pseudolarix kaempferiGord., Chenopodium ambrosioidesL., Smilax glabra Roxb., Smilax lanceaefolia Foxb. var. opaca A. DC., S. mairei Levl., S. menispermoiden DC., Alpinia japonica Miq., Cephalotaxus sinesis (Rehd. et wils.), Cephalotaxus harringtonia (Forkes) K. Koch var. harringtonia C. V. Fastigiata, Origanum vulgare L., Canpanumoea javanica B1., Berberis julianae Schneid., Berberis gagnepainii Schneid , Hudrangea strigosa Rehd., Hydrangea umbellate Rehd., Hydrangea paniculata Sieb., Discolia vittifrons Sch., Apios fortunei Maxim., wikstroemia dolichantha Diels, Collocalia inopina Thayer et Bangs., Achilea wilsoniana (Heim.) Heim., Talinum paniculatum (Jacq.) Gaertn., Rumex madaio Mak., Vaccinium fragile Franch., Euphorbia prolifera buch.-Ham., Hymenodictyon excelsum (Roxb.) wall., Lonicera confusa DC., Vaccinium fragile Franch., Gouania leptostachya DC., Hordeum vulgare L., Ziziphus jujuba Mill. var. inermis (Bge.) Rehd., Rheum palmatum L., Rheum tanguticum Maxim. ex Reg., Rheum officuinale Baill., Euphorbia pekinensis Rupr., Knoxia valerianoides Thorel, Euphorbia soongarica Boiss., Euphorbia pekinensis Rupr. var. japonensis Mak., Euphorbia pontica Prokh, Alliumsativum L, Allium scorodoprasum L., Cirsiumjaponicum DC., Alisma canaliculatum A. Br. et Bouche, Leibnitzia anandria (L.) Nakai, Bauhinia faberi Oliv., Rubus multibracteatus Levl. & Vant., Hydnocarpus anthelmintica Pier., Colysis henryi (Bak.) Ching., Marsdenia griffithii Hook. f. , Adenosma indanum (Lour.) Merr., Gryllus testaceus walker., wisteria venusta Rehd. et wils., Sargentodoxa cuneata (Oliv. ) Rehd. et wils., Myrsine africana L., Hordeum vulgare L., Schisandra micrantha A. C. Smith, Alpinia galanga (L.) Swartz, Heliotropium indicum L., Adhatoda vasica Nees, Clerodendron cyrtophyllum Turcz., Polygonum tinctorium Ait., Isatis tinctoria L., I. indigotica Fort., Baphicacanthus cusia (Nees) Bremek., Hygrophila salicifolia (Vahl) Nees, Clerodendron cyrtophyllum Turcz., Neolepisorus ovatus (Bedd.) Ching, Schefflera delavayi (Franch.) Harms., Ulmus tonkinensis Gagn., Alpinia speciosa K. Schum., Girardinia palmate (Forsk.) Gaud., Mussaenda erosa Champ., Euphorbia nematocypha Hand.-Mazz., Pistia stratiotes L., Rheum palmatum L., R. tanguticum Maxim. ex Reg., R. officinale Baill., Heteropanax fragrans (Roxb.) Seem., Dolichos falcatus Klein, Pouzolzia sanguinea (B1.) Merr., Parthenocissus laetevirens Rehd., Vicia sativa L., Inula pterocaula Franch., Blumea densiflora (Heyne) DC., Areca catechu L., Musa paradisiacal L. var. sapientum O. Ktze., Indigofera tinctoria L., Lycoris aurea Herb., Didissandra sesquifolia C. B. Clarke, Lippia nodiflora Rich. var. sarmentosa Schou., Stylophorum lasiocarpum (Oliv.) Fedde, Ampelocissus artemisiaefolia Planch., Dobinea delavayi (Baill.) Engl., Euphorbia hirta L., Pterospermum grande Craib, Rubus setchuenensis Bur. et Franch., Anemone tomentosa (Maxim.) Pei, Caesalpinia magnifoliolata Metcalf., Pteris nervosa Thunb., Zanthoxylum dissitum Hemsl., Uncaria macrophylla wall., Mosla dianthera (Ham.) Maxim, Eucalyptus robusta Sm., Callicarpa macrophylla Vahl., Machilus leptophylla Hand.-Mazz., Senecio orgzetorum Diels, Senecio vulgaris L., Hypericum przewalskii Maxim., Cayratia oligocarpa (Levl. et Vant.) Gagn., Cruculigo capitulata (Lour.) O. Ktze., Lysimachia phullocephala Hand.-Mazz., Cirsium eriophoroideum (Hook. f.) Petrak., Solanum spirale Roxb., Chrysosplenium hydrocotylifolium Levl. et Van. , Polygala chinensis L., Polytrichum commune Hedw., Melastoma normale D. Don, Lysimachia christinae Hance, Lonicera similes Hemsl. var. delavayi (Franch.) Rehd., Setaria faberii Herrm., Sanicula lamellijera Hance,Saniculastapfiana wolff, Hostasieboldiana Engl., Bauhinia pernervosa L. Chen, Schefflera delavayi (Franch.) Harms., Endospermum chinense Benth., Gelsemium elegans Benth., Mariscus umbellatus Vahl, Pollia aclisia Hassk., Ligularia lapathifolia (Franch.) Hand.-Mazz., Vaccinium dunaliamum wight. var. urophyllum Rehd. et wils., Gaultheria forrestii Diels, Polistes mandarinus Sauss., Torricellia angulata Oliv. var. intermedia (Harms) Hu, Hydrocotyle javanica Thunb. var. chinensis Dunn ex Shan et Liou, Rhynchoglossum obliquum Bl.,Elsholtzia penduliflora w.w.Smith,Stellaria paniculigera Mak.,Mallotusbarbatus (wall.) Muell.-Arg., Thalictrum faberi Ulbr., Anaphalis margaritacea (L.) Benth. et Hook. f., Stenoloma chusanum (L.) Ching, Ophiopogon dracaenoides (Bak.) Hook. f., Uncaria macrophylla wall., Indigofera teysmannii Miq., Isodon nervosus (Hemsl.) Kudo, Euonymus grandiflorus wall., Bauhinia pernervosa L. Chen, Chonemorpha megacalyx Pier., Clematis argentilucida (Levl. et Vant.) w. T. wang, Moghania macrophylla (willd.) O. Ktze., Zanthoxylum dissitum Hemsl., Indigofera teysmannii Miq., Moghania bracteata (Roxb.) Li, Crepis lignea (Van.) Babc., Lycopodium pulcherrimum wall., Tagetes erecta L., Tagetes minuta (T. glanduliflora), Rohdea japonica Roth, Crepis lignea (Van.) Babc., Viola diamantiaca Nakai, Clinopodium megalanthum (Diels) C.' Y. wu et Hsuan, Ampelopsis bodinieri (Levl. et Vant.) Rehd., Lepidogrammitis rostrata (Bedd.) Ching, Lilium concolor Salisb., Gallus gallus jabouillei Delacour et Kinnear, Dioscorea opposita Thunb., Dioscorea japonica Thunb., D. deltoidea wall., Kaempferia galanga L., Allium japonicum Reg., Alpinia japonica Miq. , Crataegus pinnatifida Bge., Crataegus cuneata Sieb. et Zucc., Crataegus pinnatifida Bge., C. hupensis Sarg., C. sanguinea Pall., C. scabrifolia (Franch.) Rehd., Crataegus oxyacantha, C. curvisepalaz, C. monogyna, Allium nipponicum Fr. et Sav., Piper hancei Maxim., Melodinus suaveolens champ. ex Benth., Geastrum hygrometricum Pers., Psychotria rubra (Lour.) Poir., Papaver nudicaule L. subsp. Amurense N. A. Busch, Rheum franzenbachii Münt., Glycosmis citrifolia (willd.) Lindl., Desmodium racemosum (Thunb.) DC., Clematis finetiana Levl. et Vant., Lilium concolor Salisb., Mussaenda pubescens Ait. f., Mussaenda parviflora Miq., Aster ageratoides Turcz., Indigofera szechuenensis Craib., Helicteres angustifolia L., Asparagus lycopodineus wall. ex Beker, Cinnamomum obtusifolium (Roxb.) Nees, Garcinia multiflora Champ., Naemorhedus goral Hardwicke, Salix tetradenia Hand.-Mazz., Sophora subprostrata Chun et T. Chen, Menispermum daurteum DC., Indigofera fortunei Craib , I. amblyantha Craib, I. ichangensis Craib, I. carlesii Craib, I. potaninii Craib, I. kirilowii Maxim. et Palib., Dunbaria circinalis (Benth.) Bak., Beesia calthaefolia (Maxim.) Ulbr., Osyris wightiana wall. ex wight, Pyrus pashia Buch.-Ham. ex D. Don, Argyreia seguinii (Levl.) Vaniot, Michelia yunnanensis Franch., Codonopsis convolvulacea Kurz. var. typical Anthony, Capricornis sumatraensis Bechstein, Pittosporum glabratum Lindl., Ilex franchetiana Loes., Juniperus taiwaniana Hayata, Rosa sericea Lindl., Ixeris chinensis (Thunb.) Nakai, Lactuca elata Hemsl., Symplocos caudate wall., Symplocos racemosa Roxb. , Celastrus stylosus wall. ssp. glaber D. Hou, Trema dielsiana Hand. -Mazz., Trema angustifolia Bl., Hieracium krameri Fr. et Sav., Lindera glauca (Sieb. et Zucc.) Bl., Carlesia sinensis Dunn, Cornus officinalis Sieb. et Zucc., Camellia japonica L., Dioscorea opposita Thunb., Allium bulleyanum Diels , Isodon pubescens (Hemsl.) C. Y. wu et Hsuan, Alpinia japonica Miq., Cassia mimosoides L., Polygala wattersii Hance, Mallotus nepalensis Muell.-Arg., Carya cathayensis Sarg., Millettia speciosa Champ., Lactuca indica L., Begonia yunnanensis Levl.,Codonopsis lanceolata Benth. et Hook., Lobelia sessilifolia Lamb., Tricyrtis pilosa wall., Clausena excavata Burm. f., Dianella ensifolia (L.) DC., Alchornea davidi Franch., Psilopeganumsinense Hemsl., Zingiber mioga Rose., Pellionia minima Mak., Zanthoxylum dissitum Hemsl., Clitoria hanceana Hemsl., Crataegus pinnatifida Bge. var. major N. E. Br., C. cuneata Sieb. et Zucc., Crataegus oxyacantha, C. monoguna, Carex baccans Nees, Rhodomyrtus tomentosa (Ait.) Hassk., Cremastra variabilis (Bl.) Nakai, Pleione yunnanensis (Rolfe) Rolfe, Oreorchis patens (Lindl.) Lindl., O. foliosa Lindl., Orthosiphon wulfenioides (Diels) Hand.-Mazz., Rhus delavayi Franch., Prunus tomentosa Thunb., Fortunella hindsi (Champ.) Swingle, Dracocephalum moldavicum L., Lindera reflexa Hemsl.; Teucrium viscidum Bl., Teucrium scorodonia, Gutzlaffia aprica Hance, Psychotria rubra (Lour.) Poir., Vigna vexillata (L.) Benth. var. yunnanensis Franch., Vigna vexillata Benth., Viburnum foetidum wall. var. ceanothoides (C. H. wright) Hand.-Mazz., Fimbristylis fusca (Nees) Benth., Sapium discolor (Champ.) Muell.-Arg., Mussaenda pubescens Ait. f., Musa paradisiacal L. subsp. Seminifera (Lour.) K. Schum., Evodia trichotoma (Lour.) Pier., Ilex franchetiana Loes., Acronychia pedunculata (L.) Miq., Lindera glauce (Sieb. et Zucc.) Bl., Cudrania cochinchinensis (Lour.) Kudo et Masam., Cassia mimosoides L., Pleomele cambodiana (Gagn.) Merr. et Chun, Cardiocrinum giganteum (wall.) Mak., Rhynchosia dielsii Harms, Vicia amoena Fisch. , Vicia cracca L. , V. pseudo-orobus Fisch. et Mey., Artemisia cina Berg., Cremastra variabilis (Bl.) Nakai, Pleione bulbocodioides (Franch.) Rolfe, Prunus tomentosa Thunb., Swertia punicea Hemsl., Vitis amurensis Rupr., Acronychia pedunculata (L.) Miq., Philadelphus sericanthus Koehne, Gomphrena globosa L., Moghania philppinensis (Merr. et Rolfe) Li, Lindera chunii Merr., Homalomena occulta (Lour.) Schott, Senecio scandens Buch.-Ham., Lycopodium serratum Thunb., Lycopodium serratum Thunb. var. thunbergii, Euphorbia lathyris L., E. amygdaloides, Eupatorium fortunei Turcz. , Stephania japonica (Thunb.) Miers, Lythrum salicaria L., Lythrum anceps Mak., Tinospora crispa (L.) Miers, Stellaria yunnanensis Franch., Semiaquilegia adoxoides (DC.) Mak., Ligusticum wallichiiFranch.,Cnidium officinala Mak., Clematis armandi Franch., Clematis Montana Buch.-Ham., Clematis apiifolia DC., C. henryi Oliv., C. pavoliniana Pamp., C. lesehenaultiana DC., C. armandi Franch. var. biondiana (Paval.) Rehd., C. gouriana Roxb. Var. finetii Rehd. et wils., Fritillaria cirrhosa D. Don, Fritillaria cirrhosa D. Don var. ecirrhosa Franch., Fritillaria delavayi Franch., Fritillaria przewalskii Maxim, Fritillaria ussuriensis Maxim., Fritillaria pallidiflora Schreb., Fritillaria maximowiczii Freyn, Fritillaria roylei Hook., Cyathula officinalis Kuan, Cyathula capitata (wall.) Moq., Aconitum carmichaeli Debx., Aconitum japonicum Nakai, Ligusticum brachylobum Franch., Cheilanthes chusana Hook., Melia toosendan Sieb. et Zucc., Melia azedarach L., Chloranthus serratus (Thunb.) Roem.et Schult., Serratula chinensis S. Moore, Eupatorium chinense L., Desmodium styracifolium (Osbeck) Merr., Clematis apiifolia DC., Aster fastigiatus Fisch., Rhamnus heterophylla Oliv., Ligustrum lucidum Ait. , Melandrium apricum (Turcz.) Rohrb., Berchemia yunnanensis Franch., Triticum aestivum L., Lysimachia deltoidea wight var. cinerascems Franch., Polygala tenuifolia willd., Allium scorodoprasum L., Cephalanoplos segetum (Bge.) Kitam., Cephalanoplos setosum (Bieb.) Kitam., Berberis amurensis Rupr., Berberis poiretii Schneid., Berberis thunbergii DC., Berberis wilsonae Hemsl., B. sibirica Pall., B. anhweiensis Ahrendt., B. subacuminata C. K. Schneid., B. vulgaris L., Polygala sibirica L. var. megalopha Fr., Codonopsis cardiophylla Diels, Veratrum mengtzeanum Loesen. f., Clematis lasiandra Maxim., Millettia pulchra Kurz var. typical f. laxior Dunn., Leontopodium japonicum Miq., Pyrrosia assimilis (Bak.) Ching, Salix hypoleuca Seem., Morus australis Poir., Aconitum bullatifolium Levl.,Aconitum pulchellum Hand.-Mazz., Tylophora yunnanensis Schltr., Lindernia mumularifolia (D. Don) wettst., Asparagus officinalis L., Pittosporum floribundum wight et Arn., Schisandra propinqua (wall.) Baill. var. sinensis Oliv., Actinidia purpurea Rehd., Euphrasia regeliiwettst., Lyonia ovalifolia (wall.) Drude, Polygonum chinense L. var. his Rubia yunnanensis (Franch.) Diels, Parthenocissus himalayanaa (Royle) Planch., Triticum aestuvum L., Micromelum integerrimum (Ham.) Roem., Hypericum erectum Thunb., Ardisia punctata Lindl., Populus pseudo-simoniiKitag., Inula cappa DC., Rosa cymosa Tratt., Jasminum f loridum Bge., Breynia patens (Roxb.) Benth., Leptadina retticulata, Delphinium yunnanense Franch., Delphinium delavayi Franch., Crotalaria szemoensis Gagn., Stachyurus himalaicus Hook. f.et Thoms., Stachyurus chinensis Franch., S. yunnanensis Franch., S. obovatus (Rehd.) Li, S. retusus Yang, S. szechuanense Fang, S. salicifolius Franch., Spilanthes callimorpha A. H. Moore, Vicia hirsute (L.) S. F. Gray, Polygonum plebeium R. Br., Cladonia verticillata Hoffm., Sanicula astrantifolic wolff ex Kreschmer, Aleuritopteris kuhnii (Milde) Ching, Sedum bulbiferum Mak., Haloragis micrantha R. Br., Tetrastigma obtectum (wall.) Pl. var. glabrum Gagn., Berberis wilsonae Homsl., Sinocrassula sp., Euphorbia thymifolia L., Arenaria serpyllifolia L'., Rubus parkeri Hance, Asplenium pekinense Hance, Gentiana rhodantha Franch., Photinia parvifolia (Pritz.) Schneid, Rhododendron capitatum Maxim., Rhododendron anthopogonoides Maxim., Agapetes mannii Hemsl., Morus australis Poir., Ziziphora clinopodioides Lam., Striga masuria (Buch.-Ham.) Benth., Selaginella davidii Franch., Lysimachia congestifloa Hemsl., Gardneria angustifolia wall., Boehmeria spicata (Thunb.) Thunb., Sopubia trifida buch.-Ham., Cyclea racemosa Oliv., Eragrostis Poaeoides Beauv., Veronica javanica Bl., Oreocharis flavida Merr., Dichondra repens Forst., Rosa cymosa Tratt. , Aletris spicata (Thunb.) Franch., Aletris glabra Bureau et Franch., Breynia patens (Roxb.) Benth., Cucubalus baccifer L., Lespedeza tomentosa (Thunb.)Sieb., Lespedeza capitata, Ampelopsis humulifolia Bge, Lycopodium selago L., Iris collettii Hook. f., Tripterospermum coeruleum (Hand.-Mazz. ex H. Sm.) H. Sm., Clematis gouriama Roxb. var. finetii Rehd. et wils., Croton lachnocarpus Benth., Dasiphora parvifolia (Fisch.)Juz., Onychium japonicum (Thunb.) Kunze, Isodon amethystoides (Benth.) C.Y. wu et Hsuen, Carpinus cordata Bl. Var. chinensis Franch., Cardiospermum halicacabum L. var. microcarpum (Kunth) B1., Dryoathyrium okuboanum (Mak.) Ching, Carduus crispus L., Carduus acanthoides L., Eupatorium odoratum L., Cynanchum auriculatum Royle ex wight, Consolida ajacis (L.) Schur, Delphinium elatum, Pothos repens (Lour.) Merr., Cyathea spinulosa wall., Toddalia asiatica (L.) Lam., Stellaria dichotoma L., Equus caballus (L.), Kalimeris indica (L.) Schulz-Bip., Equus caballus (L.), Prospirobolus joannsi (Brolemann), Equus caballus (L.), Lasiosphaera fenzlii Reich., Calvatia gigantean (Batsch ex Pers.) Lloyd, Calvatia lilacina (Mont. & Berk. ) Lloyd, Lycoperdon gemmatum Batsch., Setaria plicata (Lamk.) T Cooke, Equus caballus (L.), Digitaria sanguinalis (L.) Scop., Equus caballus (L.), Solen gouldii Conrad, Nothapodytes pittosporoides (Oliv.) Sleum., Calanthe fimbriata Franch., Pedicularis resupinata L., Thalictrum foliolosum DC., Thalictrum baicalense Turcz, Thalictrum foetidum L., T. cultratum wall., T. chaotungensis w. T. wang et S. H. wang, T. baicalense Turcz. Var. megalostigma Boivin, Hydrocharis asiatica Miq., Sambucus buergeriana B1., Portulaca oleracea L., Embelia oblongifolia Hemsl., Strychnos nux-vomica L., Strychnos pierriana A. w. Hill, Coriaria sinica Maxim., Aristolochia contorta Bge., Melothria indica Lour., Butea suberecta (Dunn) Blatter, Butea suberba, B. frondosa, Nephrolepis cordifolia (L.) Presl, Capparis pterocarpa Chun, Capparis masaikai Levl.,Capparis versicolor Griff, Iris pallasii Fisch. var. chinensis Fisch., Iris halophila Pall., Rhododendron delavayi Franch., Ipomoea pes-caprae (L.) Sweet, Cotoneaster amoena wils., Caltha palustris L., Caltha scaposa Hook. f. et Thoms., Equus caballus (L.), Caltha palustris L. var. membranacea Turcz., Angiopteris fokiensis Hieron., Verbena officinalis L., Camptosorus sibiricus Rupr., Equus caballus (L.), Sedum emarginatum Migo, Sedum makinoi Maxim., Hydrangea davidii Franch., Smilax nipponica Miq., Portulaca oleracea L., Coriaria sinica Maxim., Ipomoea pes-caprae (L.) Sweet, Saussurea cordifolia Hemsl., Lycopodium sieboldii Miq., Trichosanthes cucumeroides (Ser.) Maxim., Trichosanthes cucumeroides Maxim., Thladiantha dubia Bge., Paris tetraphylla A. Gray, Trichosanthes cucumeroides (Ser.) Maxim., Vacaria segetalis (Neck.) Garcke, Melandrium apricum (Turcz.) Rohrb., Vaccaria segetalis (Neck.) Garcke., Pteris laeta (wall.) Ching, Anoplophora chinensis (Frster), Apriona germari (Hope), Allium funckiaefolium Hand.-Mazz., Gastrodia etata Bl., Semiaquilegia adoxoides (DC.) Mak., Asparagus cochinchinensis (Lour.) Merr., Asparagus filicinus ex D. Don Ham., A. meioclados Levl.,A. spinasissimus wang et S.C. Chen, Asparagus racemosus, Triosteum pirinatifidum Maxim., Spilanthes acmella (L.) Murr., Diuranthera minor (C. H. wright) Hemsl., Hyoscyamus niger L.,Hygrophilasalicifolia (Vahl)Nees,Aristolochia debilisSieb. et Zucc., Aristolochia contorta Bge., Carpesium abrotanoides L., Trichosanthes kirilowii Maxim., Solanum indicum L., Physalis minima L., Hydrocotyle sibthorpioides Lam., Arisaema consanguineum Schott, Arisaema amurense Maxim., Arisaema Heterophyllum B1., Arisaema ambiguum Engl., Arisaema peninsulae Nakai, Arisaema thunbergii B1., Arisaema verrucosum Schott, Arisaema lobatum Engl. Var. rosthornianum Engl., Arisaema elephas S. Buchet, Osbeckia chinensis L., Metaplexis japonica (Thunb.) Mak., Luffa cylindrical (L.) Roem., Aucuba chinensis Benth., Gastrodia elata Bl., Semiaquilegia adoxoides (DC.) Mak., Stellaria alsine Grimm., Sorbus tianschanica Rupr., Triosteum pinnatifidum Maxim., Gnaphalium hypoleucum DC., Magnolia amoena Cheng, Magnolia cylindrical wils., Gnaphaliumjaponicum Thunb., Fagopyrum cymosum Meisn., Aucuba chinensis Benth., Gastrodia elata B1., Triosteum pinnatifidum Maxim., Litsea auriculata Chien et Cheng, Cassytha filiformis L., Pistacia vera L., Pyrolusite, Ficus carica L., Ampelopsis cantoniensis (Hook. et Arn.) Planch., Sapindus mukorossi Gaertn., Phoenix dactylifera L., Pistacia vera L., Ficus carica L., Sapindus mukorossi Gaertn., Prunus brachypoda Batal. var. eglandulosa Cheng, Scutellaria indica. L. var. humilis Mak., HypericumsampsoniiHance, Musxovite, Caesalpinia sepiaria Roxb., Caesalpinia bonducella, Achyranthes aspera L. var. rubrafusca (wight) Hook., Caesalpinia sepiaria Roxb., elphinium giraldii Diels, Caesalpinia sepiaria Roxb., Rubia yunnanensis (Franch.) Diels, Asbestus, Chaenomeles lagenaria (Loisel.) Koidz., Chaenomelessinensis (Thouin) Koehne, C. lagenaria (Loisel.) Koidz. var. Cathayensis Rehd., C. lagenaria (Loisel.) Koidz. var. wilsonii Rehd., C. thibetica Yw, Auricularia auricula (L.ex Hook.)Underw., Cajanus cajan (L.) Millsp., Saussurea lappa Clarke, Vladimiria denticulata Ling, Vladimiria souliei (Franch.) Ling, Vladimiria muliensis (Hand.-Mazz.) Ling, V. edulis (Franch.) Ling, Schima superba Gardn. et Champ., Equisetum Hiemale L., E. arvense L., Akebia trifoliata (Thunb.) Koidz. var. custralis (Diels) Rehd., Akebia trifoliata (Thunb.) Koidz. , Akebia quinata (Thunb.) Decne., Aristolochia manshuriensis Kom., Clematis armandi Franch., C. montana Buch.-Ham., Aristolochia kaempferi willd., A. moupinensis Franch., Akebia quinata (Thunb.) Decne., Akebia longeracemosa Matsum, Indigofera tinctoria L., Actinidia polygama (Sieb. et Zucc.) Miq., Chaenomeles lagenaria (Loisel.) Koidz., Magnolia liliflora Desr., Elaeagnus multiflora Thunb., Garcinia oblongifolia Champ., Cajanus cajan (L.) Millsp., Litsea pungens Hemsl., Litsea cubeba (Lour.) Pers., L. euosma w. w. Smith, Elsholtzia cypriani (Pamp.) C. Y. wu et S. Chow., Manglietia fordiana (Hemsl.) Oliv., Akebia quinata (Thunb.) Decne., A. trifoliata (Thunb.) Koidz., A. trifoliata (Thunb.) Koidz. var. australis (Diels) Rehd., Mahonia shenii Chun, Mahonia schochii Schneid. ex Hand.-Mazz., M. subimbricata Chun et F. Chun, M. taronensis Hand.-Mazz., Litsea pungens Hemsl., Gomphostemma microdon Dunn, Gossampinus malabarica (DC.) Merr., Ficus pumila L., Hibiscus syriacus L., Oroxylum indicum (L.) Vent., Momordica cochinchinensis (Lour.) Spr., Actinidia polygama (Sieb. et Zucc.) Miq., Elaeagnus multiflora Thunb., Garcinia oblongifolia Champ., Hibiscus mutabilis L., Litsea pungens Hemsl., Viburnum macrocephalum Fort., Viburnum opulus, V. prunifolium, Oroxylum indicum (L.) Vent., Lespedeza buergeri Miq., Pseudostellaria heterophylla (Miq.) Pax ex Pax et Hoffm., Pseudostellaria Maximowicziana (Franch. et Savat.) Pax ex Pax et Hoffm., Rubus pacificus Hance, Tanacetum variifolium (Chang) Ling, Cladonia alpestris (L.) Rabht., Pedicularis davidii Franch., Pedicularis rudis Maxim., Pedicularis dunniana Bonati, Peicularis decora Franch., Aster flaccidus Bge., Plagiopus oederi (Brid.) Limpr., Tetraplodon bryoides (Zoeg.) Lindb., Tongoloa dunnii (Boiss.) wolff, Fritillaria taipaiensis P. Y. Li, Cladonia gracilis (L.) willd., Cremanthodium hookerii Clarke,Rubusblinii Levl., Ipomoea cairica (L.) Sweet, Antidesma bunius (L.) Spr., Anaphalishancockii Maxim., Ficus simplicissima Lour. var. hirta (Vahl.) Migo, Rubus cochinchinensis Tratt., Acanthopanax gracilistylus w. w. Smith, A. sessiliflorus (Rupr. et Maxim.) Seem., A. senticosus (Rupr. et Maxim.) Harms., Acanthopanax henryi (Oliv.) Harms, Acanthopanax verticillatus Hoo, Acanthopanax giraldii Harms, A. trifoliatus (L.)Merr., A. evodiaefolium Franch. var. ferrugineus w. w. Smith, A. setchuenensis Harms, A. leucorrhizus (Oliv.) Harms, Periploca sepium Bge., Lantana camara L., Chrysomyia megacephala (Fab.), Trogopterus xanthipes Milne-Edwards, Triosteum fargesii Franch., Schisandra chinensis (Turcz.) Baill., Schisandra sphenanthera Rehd. et wils., Corydalis stenantha Franch., Echinopsilon divaricatum Kar. et Kir., Mosla soochouensis Matsuda, Schisandra propinqua (wall.) Bail. var. intermedia A. C. Smith, Evodia trichotoma (Lour.) Pier., Rhus chinensis Mill., R.potaninii Maxim., R. punjabensis Stew. var. sinica (Diels) Rehd. et wils., Melaphis chinensis (Bell), Melaphis paitan Tsai et Tang, Tetrastigma hypoglaucum Planch., Lantana camara L., Acer sinense Pax,. Acer sinopurpurascens Cheng, A. henryi Pax, A. palmatum Thunb., Abelmoschus sagittifolius (Kurz) Merr., Kadsura longipedunculata Finet, Schisandra sphenanthera Rehd. et wils., Kadsura coccinea (Lem.) A. C. Smith, Schisandra propinqua (wall.) Hook f. et Thoms, Rhus chinensis Mill., Lorahthus pentapetalus Roxb., Geum aleppicum Jacq., Ipomoea cairica (L.) Sweet, Pedicularis rex C. B. Clarke ex Maxim., Vitis quinquangularis Rehd., Ficus simplicissima Lour., Melaphis chinensis (Bell), M. paitan Tsai et Tang, Choerospondias axillaries (Roxb. ) Burtt et Hill, Meconopsis quintuplinervia Reg., Lepisorus thunbergianus (Kaulf.) Ching, Orostachys fimbriatus (Turcz.) Berger, Orostachys erudescens (Maxim.) Ohwi, Orostachys spinosus (L.) C. A. Mey., Melandryun viscidulum (Franch.) Hand.-Mazz. var. szechuanense (wills.) Hand.-Mazz., Arca inflata Reeve, A. granosa L., A. subcrenala Lischke, Plantago asiatica L., Plantago depressa willd., Plantago major L., Plantago ovata, Dodonaea viscose (L.) Jacq., Oldenlandia uncinella (Hook. etArn.) O. Ktze. var. scabrida (Franch.) Chun, Cynoglossum lanceolatum Forsk., Gynura sagittata DC., Holarrhena antidysenterica wall., Fimbristylis miliacea (L.) Vahl, Monetaria moneta (L.), Monetaria annulus (L.), Coelogyne corymbosa Lindl., Zanthoxylum dimorphophyllum Hensl. var. spinifolium Rehd. et wils., Liparis nervosa (Thunb.) Lindl., Anaphalis bulleyana (J.F.Jaffr.) Hand.-Mazz., Bos taurus domesticus Gmelin, Bubalus bubalis L., Aconitum ochranthum Mey., Achyranthes bidentata B1., Mucuna sempervirens Hemsl., Oreas martiana (Hopp. et Hornsch.) Brid., Heleocharis yokoscensis (Franch. et Savat.) Tang et wang, Oldenlandia hedyotidea (DC.) Hand.-Mazz., Elaeagnus umbellate Thunb., Ficus hispida L. f., Ficus beecheyana Hook. et Arn., Rumex patientia L., Didymocarpus eburneus (Hance) Levl.,Boea hygrometrica (Bge.) R. Br., Sonchus arvensis L., Rumex dentatus L., Sida retusa L., Lygodium flexuosum (L.) Sw., Dodartia orientalis L., Rubus ichangensis Hemsl. et O. Ktze., Smilax riparia A. DC., Circaea cordata Royle, Alnus lanata Duthie, Eleusine indica (L.) Gaertn., Arctium lappa L., Fortunearia sinensis Rehd. et wils., Cerbera manghas L., Oldenlandia hedyotidea (DC.) Hand.-Mazz., Ficus hispida L.f., Ficus beecheyana Hook. et Arn., Rumex crispus L., Rumex nepalensisSpr., Daphniphyllum calycinum Benth., Arctium lappa L., Saurauia lantsangensis Hu, Saurauia napaulensis DC., Achyranthes bidentata Bl., Rumex crispus L., Aleurites fordii Hemsl., Gymnadenia conopsea R. Br., Gymnadenia crassinervis Finet, Coeloglossum viride (L.) Hartm. var. bracteatum (willd.) Richter, Ranunculus japonicus Thunb., Lycianthes biflora (Lour.) Bitter, Phyllostachys pubescens Mazel ex H. de Lehaie, Melastoma sanguineum Sims, Polygonum barbatum L., Dioscorea alata L., Clematis argentilucida (Levl. et Vant.) w. T. wang, Populus tomentosa Carr., Actinidia eriantha Benth., Ilex pubescens Hook. et Arn., Ilex pubescens Hook. et Arn. var. glabra Chang, Picris hieracioides L. subsp. fuscipilosa Hand. -Mazz., Ardisia pusilla DC., Dryopteris championi (Benth.) C. Chr. ex Ching, Adonisszechuenensis Franch., Verbascum thapsus L., Adenosma glutinosum (L.) Druce, Dysosma hispida (Hao) Chun, Gerbera piloselloides Cass., Philadelphus henryi Koehne, Croton caudatus Geisel. var. tomentosus Hook., Nanocnide pilosa Migo, Cornus walteri wanger., Desmodium elegans (Lour.) Benth., Gerbera piloselloides Cass., Lindera caudate (wall.) Benth., Photinia villosa (Thunb.) DC., Meconopsis quintuplinervia Reg., Leptodermis pilosa (Franch.) Diels, Geranium eriostemon Fisch., Cimicifuga foetida L., Cimicifuga dahurica (Turcz.) Maxim., Cimicifuga heracleifolia Kom., Serratula chinensis S. Moore, Aruncus Sylvester Kostel., Anhydrite, Lepisorus psedonudus Ching, Arundina chinensis Bl., Seseli giraldii Diels., Catharanthus roseus (L.) G. Don., Catharanthus roseus (L.) G. Don var. albus (Sweet) G. Don, C. roseus (L.) G. Don var. flavus (Tsiang) Metcalf, Vinca minor L., Vinca erecta Rgl. et Schmach, Platanthera japonica (Thunb.) Lindl., Callicarpa loureiri Hook. et Arn., Helicteres elongata wall., Pedicularis longiflora Rudolph. var. tubiformis (Klotz.) Tsoong, Clerodendron indicum (L.) O. Ktze., Cymbidium faberi Rolfe, Melasma arvense (Benth.) Hand.-Mazz., Crotalaria tetragona Roxb., Citrus grandis (L.) Osbeck var. tomentosa Hort., Citrus grandis (L.) Osbeck, Platycarya strobilacea Sieb. et Zucc., Platycarya longipes wu, Schizothorax yunnanensis Norman, Juglans regia L., Lindera strychnifolia (Sieb.etZucc.) Villar, Corvus macrorhynchus wagler, Prunus mume (Sieb.) Sieb. et Zucc., Zaocys dhumnades (Cantor), CanariumpimelaKoenig, Tarenna mollissima (Hook. et Arn.) Robins., Diospyros ebenum Koen., Sophora mairei Pamp., Vaccinium fragile Franch.,Swertia heterantha Ling, SepiellamaindronideRochebrune, Sepia esculenta Hoyle, Salix microstachya Turcz., Lindera strychnifolia (Sieb. et Zucc.) Villar, Clerodendron yunnanense Hu ex Hand.-Mazz., Corvus macrorhynchus wagler, Gallus gallus domesticus Brisson, Fissistigma glaucescens (Hance) Merr., Sapium sebiferum (L.) Roxb., Periploca calophylla (wight) Falc., Canarium pimela Koenig, Cayratia japonica (Thunb.) Gagn., Cayratia japonica (Thunb.) Gagn. var. pubifolia Merr. et Chun, Viola vaginata Maxim., Rubus tephrodes Hance, Gentiana urnula H. Sm., Corvusmacrorhynchus wagler, Sepiella maindronide Rochebrune, Sepia Esculenta Hoyle, Oenothera erythrosepala borb., Rosa chinensis Jacq., Laurus nobilis L., Salvia miltiorrhiza Bge., Salvia przewalskii Maxim., S. przewalskii Maxim. var. mandarinorum (Diels) Stib., S. yunnanensis C. H. wight, Ficus martini Levl. et Vant., Dioscorea japonica Thunb., Hiptage benghalensis (L.) Kurz, Adenostemma lavenia (L.) O. Ktze., Rorippa palustris (Leyss.) Bess., Clinopodium chinense (Benth.) O. Ktze., Cephalanthus occidentalis L., Clematis chrysocoma Franch., Impatiens balsamina L., Rhodiola dumulosa (Franch.), Pedicularis henryi Maxim., Pedicularis longicaulis Franch. et Maxim., Pteris multifida Poir., Elsholtzia bodinieri Van't, Pteris ensiformis Burm., Sterculia nobilis Smith, Ailanthus altissima (Mill.) Swingle, Gallus gallus domesticus Brisson, Polystichum acanthophyllum (Fr.) Bedd., Cycas revolute Thunb., Sterculia nobilis Smith., Asplenium yunnanense Franch., Arthromerismairei (Brause) Ching, Caryopteris terniflora Maxim., Viburnum propinquum Hemsl., Rhododendrom molle (Bl) G. Don, Iris speculatrix Hance, Eupatorium chinense L., Asparagus plumosus Bak., Meretrix meretrix L., Xanthoceras sorbifolia Bge., Cypsilurus agoo (Temm. et Schl.), Crinum asiaticum L., var. sinicum Bak, Calcite, Sus scrofa domestica Brisson, Helicteres isora L., Euphorbia antiquorum L., Cannabis sativa L., Aconitum sungpanense Hand.-Mazz., PolygonumchinenseL., P. chinensevar. thunbergianum, Streptocaulon griffithii Hook. f., Euphorbia antiquorum L., Tupistra pachymena wang et Tang, Epilobium pyrricholophum Franch. et Sav., Viburnum cordifolium wall. et DC., Croton tiglium L., Solanumindicum L., Miscanthus floridulus (Labill.) warb., Morinda officinalis How, Morinda citrifolia, M. tictoria , Morinda lucida, Prunus amygdalus Batsch., Prunus amygdalus Batsch var. dulcis Schneider, P. amygdalus Batsch var. amara Schneider, Triplostegia grandiflora Gagn., Habenaria dentate (Sw.) Schltr., Microtis taiwaniana Schltr., Bauhinia hupehana Craib, Aubskuaea carduiophylla Franch., Elsholtzia fruticosa (D. Don) Rehd., Ceropegia dolichophylla Schltr., Christia vespertilionin (L. f.) Bahn. F., Asplenium sarelii Hook., Tagetes patula L., Oenanthe j avanica (B1.) DC., Stachys baicalensis Fisch., Stachys recta L., Stachys neglecta Klok., Stachys betonicaeflora, Codium fragile (Sur.) Har., Solanum torvum Sw., Hydrotrechus remigator Hor., Gardenia jasminoides Ellis var. grandiflora Nakai, Gardenia jasminoides Ellis var. radicans Mak., Enhydris chinensis (Gray), Cinnabar, Mercury, Scirpus validus Vahl, Hirudo nipponia whitman, whitmania Pigra (whitman), whitmania acranulata (whitman), whitmania edentula (whitman), Polygonum hydropiper L., Ceratopteris thalictroides (L.) Brongn., Ludwigia prostrata Roxb., Begonia pedatifida Levl., Mnium cuspidatum Hedw., Bubalus bubalis L., Polypodium niponicum Mett., Polypodium vulgare L., P. pseudoamoenum Ching, Tacca plantaginea (Hance) Prenth., Tacca chantrieri Andre, Boehmeria grandifolia wedd., Narcissus tazetta L. var. chinensis Roem., Jussiaea suffruticosa L., Narcissus papyraceus Ker-Gaw, Ligustrum quihoui Carr., Ligustrum sinense Lour. var. nitidum Rehd., Cardiocrinum cathayanum (wils.) Mak., Adina pilulifera (Lam.) Franch., Murdannia triguetra (wall.) Brcckn.,Lysimachia stenosepala Hemsl., Lysimachia circaeoides Hemsl., Homonoia riparia Lour., Salix purpurea L., Geum japonicum Thunb., Adina rubella (Sieb. et Zucc.) Hance, Limnophila aromatica (Lam.) Merr., Rotala rotundifolia (Buch.-Ham.) Koehne, Ammannia baccifera L., Oenanthe benghalensis (Roxb.) Kurz, Stachys baicalensis Fisch., Gymontheca involucrata Pei, Cassia nomame (Sieb.) Kitagawa, Saurauia tristyla DC., Glyptostrobus pensilis (Lamb.) K. Koch, Veronica anagallis-aquatica L., Impatiens uliginosa Franch., Penthorum chinense Pursh, Oldenlandia corymbosa L., Myricaria germanica (L.) Desv., Clerodendron inerme (L.) Gaertn., Limnophila rugosa(Roth) Merr., Nitella expansaAllen, Cotoneaster horizontalis Decne., Cyperus glomeratus L., Cleistocalyx operculatus (Roxb.) Merr. et Perry, Pongamia pinnata (L.) Merr., Potamogeton natans L., Gardenia jasminoides Ellis var. grandiflora Nakai, Actinidia callosa Lindl. var. henryi Maxim., Polygonum thunbergii Sieb. et Zucc., Pilea notata C. H. wright, Oreocnide frutescens (Thunb.) Miq. , Anemone hupehenis Lem. f. alba w. T. wang, Inula helianthus-aquatilis C. Y. wu ex Ling, Eichhornia crassipes Solms, Lactuca raddeana Maxim. var. elata (Hemsl.) Kitam., Monotropa uniflora L., Chloranthus fortunei (A.Gray) Solms, Neosalanx tankankei taihuensis Chen, Sorbus alnifolia (Sieb. et Zucc.) K. Koch, Kyllinga brevifolia Rottb., wendlandia uvariifolia Hance, Sagittaria aginashi Mak., Apluda mutica L., Polygonum hydropiper L., Ficus stenophylla Hemsl., Ligularia sp., Isodon adenanthus (Diels) Kudo, Tacca plantaginea (Hance) Prenth., Polygonum orientale L., P. lapathifolium L., P. lapathifolium L. var. salicifolium Sibth., Salixpurpurea L., Geum japonicum Thunb., Adina rubella (Sieb. et Zucc.) Hance, Phragmites karka (Retz.) Trin., Adina pilulifera (Lam.) Franch., Cassia nomame (Sieb.) Kitagawa, Saurauia tristyla DC., Veronica anagallis-aquatica L., Clerodendron inerme (L.) Gaertn., Lindernia antipoda (L.) Alston, Hymenocallis americana Roem., Hymenocallis speciosa, Polygala caudate Rehd. etwils., Epilobiumhirsutum L. , Torenia glabra Osbeck, Inula helianthus-aquatilis C. Y. wu ex Ling, Cacalia tangutica (Maxim.) Hand.-Mazz., Halerpestes sarmentosa (Adams) Kom., Cardamine lyrata Bge., Ligustrum quihoui Carr., Salix purpurea L., Veronica anagallis-aquatica L., Impatiens uliginosa Franch., Epilobium palustre L.,Vittaria flexuosa Fee, Polygonatum odoratum (Mill.) Druce var. pluriflorum (Miq.) Ohwi, Polygonatum macropodium Turcz., Polygonatum involucratum Maxim., Polygonatum inflatum Komar., Lycopodium obscurum L., Nepherite, Stachytarpheta jamaicensis (L.) Vahl, Magnolia denudata desr., Zea mays L., Opuntia dillenii Haw, Pedilanthus tithymaloides (L.) Poit., Hosta plantaginea (Lam.) Aschers., Solanum pseudo-capsicum L., Mahonia bealei (Fort.) Carr., M. fortunei (Lind1.) Fedde, M. japonica (Thunb.) DC., Blumea balsamifera DC., Artemisia argyi Levl. et Vant., Artemisia vulgaris L., Artemisia taurica, Blumea balsamifera DC., Benincasa hispida (Thunb.) Cogn. var. chichpua How, Alternanthera sessilis (L.) DC., Arcangelisia loureiri (Pier.) Diels, Streptocaulon griffithii Hook. f., Cryptolepis buchanani Roem. et Schult., Cryptolepis sanguinolenta Schlechter, Solanum nigrum L. var. pauciflorum Liou, Montmorillonite, Fullers earth, Nardostachys chinensis Batal., Nardostachys jatamanse DC., Glycyrrhiza uralensis Fisch., Glycyrrhiza glabra L., Glycyrrhiza kansuensis Chang et Peng, Glycyrrhiza inflata Batal., Euphorbia kansui Liou, Euphorbia sieboldiana , E. sieboldianaMorr. et Decne., Brassica oleracea L. var. capiata L., B. oleracea L., Saccharum. sinensis Roxb., Dioscorea esculenta (Lour.) Burkill, Glycyrrhiza uralensis Fisch., Musa paradisiaca L. var. sapientum (L.) O. Ktze., Oxytropis kansuensis Bge., Atractylodes macrocephala Koidz., Arachniodes amabilis (Blume) Tindale, Pyrrosia lingua (Thunb.) Farw., Pyrrosia sheareri (Bak. ) Ching, Pyrrosia drakeana (Franch.) Ching, Pyrrosiapetiolosa (Christ) Ching, Pyrrosiadavidii (Gies.) Ching, Pyrrosia gralla (Gies.) Ching, Umbilicaria esculenta (Miyoshi) Minks, Limestone, Gorgonia flabellum L., Parmelia saxatilis Ach., Parmelia saxatilis var. omphalodes, Bulbophyllum radiatum Lindl., Phryganea japonica M1., Ulva lactuca L., Ulva pertusa Kjellm., endrobium nobile Lindl., Dendrobium linawianum Reichb. f., Dendrobium officinale K. Kimura et Migo, Dendrobium moniliforme (L.) Sw., Dendrobium hercoglossum Reichb. f, Dendrobium aduncum wall. et Lindl., Dendrobium wilsonii Rolfe, Dendrobium hancockii Rolfe, Dendrobium lohohense Tang et wang, Dendrobium loddigesii Rolfe, Dendrobiumbellatulum Rolfe, P590,,, Lycorisradiata (L'Her.) Herb., Lycoris squamigera Maxim., Mitella mitella (L.), Cypsum, Cladonia rangiferina web., Sarcocheilichthys sinensis Bleeker, Cyrtiospirifer sinensis (Graban.), Mycoblastus alpinus (Fr.) Kernst., Telphusa sp., Chiton sp., Ludisia discolor (Ker-Gawl.) A. Rich., Pyrrosia lingua (Thunb.) Farw., Salvia chinensis Benth., Goodyera procera (Ker-Gawl.) Hook., Eumeces chinensis (Gray), Eumeces elegans Boulenger, Sphenomorphus indicus (Gray), Juncus effuses L. var. decipiens Buchen. f. utilis Mak., Ranunculus sceleratus L., Gontiana squarrosa Ledeb., Pholidota chinensis Lindl., Lysionotus pauciflora Maxim., HaliotisdiversicolorReeve, Haliotis gigantea discus Reeve, Elatostema laevigatum (B1.) Hassk., Peucedanum terebinthaceum (Fisch.) Fisch. ex Turcz., Eucheuma gelatinae (Esp.) J. Ag., Chlamydoboea sinensis (Oliv.) Stapf, Lycopodium clavatum L., Lycopodium obscurum L., L. complanatum L., L. alpinum L., L. annotinum L., Pilea cavaleriei Levl., Psilotum nudum (L.) Griseb., Pothos chinensis (Raf.) Merr., Photinia serrulata Lindl., Rhododendron metternichii S. et Z., Hoya lyi Levl. , Mosla scabra (Thunb.) C. Y. wu. et H. w. Li, Sedum sarmentosum Bge., Mosla chinensis Maxim., Corallodiscus flabellata (Franch.) B. L. Burtt, Pseudosciaena crocea (Rich.), Pseudosciaena polyactis Bleeker, Lepisorus eilophyllus (Diels) Ching, Polygonum capitatum Ham. ex D. Don, Aleurites moluccana (L.) willd., Petroleum, Acorus gramineus Soland., Acorus gramineus Soland. var. pusillus Engl., Anemone altaica Fisch., Berchemia hypochrysa Schneid., Dendrobium nobile Lindl., Stereocaulon paschale Hoffm., Boenninghausenia sessilicarpa Levl.,Ficus sarmentosa Buch-Ham. ex J. E. Sm. var. henryi (King) Corner, Sulphur, Pilea plataniflora C. H. wright, Pelargonium hortorum Bailey, Zacco platypus (Schl.), Punica granatum L., Peperomia dindigulensis Miq., Sedum mingjinianum Fu, Sedum tetractinum Frd., Sinocrassula indica (Decne.) Berger, Juncus effuses L. var. decipiens Buchen. f. utilis Mak., Ranunculus sceleratus L., Vaccinium saxicolum Chun ex Sleumer, Pseudosciaena crocea (Rich.), P. polyactis Bleeker, Lindera setchuenensis Gamble, Acorus gramineus Soland., Vitex quinata (Lour.) F. N. williams, Uraria crinita Desv. var. macrostachya wall., olystichum braunii (Spenn.) Fe; P624-2, Cybister tripunctatus orientalis Gschew., P625, Gentiana scabra Bge., Gentiana triflora Pall., Gentiana manshurica Kitag., Gentiana rigescens Franch., G. rigescens Franch. var. stictantha Marquand, Gentianopsis paludosa (Munro)Ma, G. lutea L., G punctata L., Tubocapsicum anomalum (Franch. et Sav.) Mak., Solanum nigrum L., Solanum auriculatum , S. xanthocarpum, Ottelia alismoides (L.) Pers., Cypripedium henryi Rolfe, Sauropus rostratus Miq., Nuphar bornetii Levl. et Vant., Physaliastrum heterophyllum (Hemsl.) Mak., Juncus setchuensis Buchen. var. effusoides Buchen., Poa sphondylodes Trin., Gracilaria verrucosa (Huds.) Papenf., Physeter catodon L., Tubocapsicum anomalum (Franch et Sav.) Mak., Passiflora foetida L., Euphoria longan (Lour.) Stend., Ixora chinensisLam., Solanum nigrum L., Agrimonia pilosa Ledeb. var. japonica (Miq.) Nakai, Sauropus rostratus Miq., Dryobalanops aromatica Gaertn. f., Rubus sieboldi Blume., Potamogeton pectinatus L., Anaphalis nepalensis (Spr.) Hand-Mazz., Porana racemosa Roxb., Anemone hupehensis Lem., Aster dubius (Thunb.) Onno, Aster scaber Thunb., Scopolia japonica Maxim., Agriophyllum arenarium Bieb., Aster scaber Thunb., Atalantia buxifolia (Poir.) Oliv., woodwardia orientalis Sm., Artemisia finita Kitag., Blechnum orientale L., Glehnialittoralis F. Schmidt ex Miq., Nitraria sibirica Pall., Ainsliaea pertyoides Franch. var. albotomentosa Beauverd, Helwingia japonica (Thunb.) Dietr., Helwingia himalaica Clarke, Helwingia chinensis Batal., Bougainvillea glabra Choisy, Bougainvillea glabra var. sandericna, Euphorbia heterophylla L., Tilia tuan Szysz., Helwingia japonica (Thunb.) Dietr., H. himalaica Clarke, H. chinensis Batal., Pleonomus canaliculatus Faldermann, Agriotes fuscicollis Miwa, Melanotus caudex Lewis., Mecopoda elongata L.', Philydrum lanuginosum Banks, Cipangopaludina chinensis (Gray), Convolvulus arvensis L., Smithia sensitiva Ait., Turbo cornutus Solander, Arisaema ringens (Thunb.) Schott, Veronicastrum cauloptera (Hance) Yamazaki, Cissus pteroclada Hayata, Galium bungei Stend., Galium gracilens (A. Gray) Mak., Chloranthus henryi Hemsl., Lysimachia paridiformis Franch., Lysimachia trientaloides Hemsl., Caesalpinia sepiaria Roxb., Anagallis arvensis L., Japalura flaviceps Barbour et Dunn, Chloranthus multistachys Pei., Schnabelia oligophylla Hand.-Mazz., Liriodendron chinensis (Hemsl.) Sarg., Cucumis melo L. var. flexuosus Naud., C. melo L. var. conomon Mak., Zingiber officinale Rosc. , Rhus verniciflua Stokcs, Stelmatocrypton khasianum (Benth.) H. Baill., Tylorrhynchusheterochaeta Quatrefages,Eria graminifolia Lindl., Hematite, Curculigo orchioides Gaertn., Phyllostachys nigra (Lodd.) Munro var. henonis (Mitf.) Stapf ex Rendle, Pleioblastus amarus(Keng) Kengf., Echinopsis multiplex Zucc., Opuntia dillenii Haw., Opuntia monacantha Haw., P664, Scorzonera albicaulis Bge., Pyrus hondoensis Nakai et Kikuchi , Opuntia dillenii Haw., Agrimonia pilosa Ledeb. var. japonica (Miq.) Nakai, Agrimonia pilosa Ledeb. var. viscidula Kom., A. pilosa Ledeb. var. nepalensis (D. Don) Nakai, A. pilosa Ledeb., A. pilosa Ledeb. var. coreana (Nakai) Liou et Cheng, A. pilosa Ledeb. f. davurica Nakai, A. asiatica Juz., Agrimonia pilosa Ledeb. var.japonica (Miq.) Nakai, Bletilla striata (Thunb.) Reichb. f., Micromelum falcatum (Lour.) Tanaka, Atractylodes macrocephala Koidz., Euonymus tengyuehensis w. w. Smith, Vigna cylindrica (L.) Skeels, Lactuca sativa L., Angelica dahurica (Fisch. exHoffm.) Benth. et Hook. f. ex Franch. et Sav., Angelica anomala Lallem., Angelica taiwaniana Boiss., Heracleum scabridum Franch., Amaranthus viridis L., Brassica alba (L.) Boiss., Alunite , Ginkgo biloba L.,Erythroculterilishaeformis (Bleeker), Chalk, P686, Cynanchum stauntoni (Decne.) Schltr. ex Levl.,Cynanchum glaucescens (Decne.) Hand.-Mazz., Prunus mume (Sieb.) Sieb. et Zucc., Acorus calamus L., Acorus calamus L. var. angustatus, Lophura nycthemera nycthemera (L.), Artemisia sieversiana Ehrh. ex willd., Ampelopsis japonica (Thunb.) Mak., Ampelopsis meliaefolia, Cynanchum atratum Bge., Cynanchum versicolor Bge., Millettia bonatiana Pamp., Passer montanus saturatus Stejneger, Passer rutilans rutilans (Temminck), Rhodiola henryi (Diels) Fu, Ledum palustre L. var. angustum E. Busch, Melaleuca leucadendra L., Serissa serissoides (DC.) Druce, Serissa foetida Comm., Tremella fuciformis Berk., Quercus acutissima Carr., Q. aliena Bl., Inula cappa DC. , Cucubalus baccifer L., Arnebia saxatilis Benth. et Hook., Solanum lyratum Thunb., Solanum dulcamara L., Delphinium pogonanthum Hand.-Mazz., Quartz, P702, Lactuca indica L., Michelia alba DC., Pulsatilla chinensis (Bge.) Reg., Oyksatukka dagyrruca (Fisch.) Spr., Pulsatilla koreana Nakai, Pulsatilla turczaninovii Krylov et Serg., Pulsatilla ambigua Turcz. ex Pritz., Potentilla chinensis Ser., P. discolor Bge., Anemone vitifolia Buch.-Ham., A. hupehensis Lem., A. tomentosa (Maxim.) Pei, Pulsatilla nigricans, Anemone cernua, Pulsatilla nigricans, Paeonia lactiflora Pall., Populus davidiana Dode, Populus bonatii Levl.,Amomum cardamomum L., Elettaria cardamomum white et Maton, Angelica dahurica (Fisch. ex Hoffm.) Benth. et Hook. f. ex Franch. et Sav., A. anomala Lallem., A. taiwaniana Boiss., Plumbago zeylanica L., Ternstroemia gymnanthera (wight et Arn.) Sprague, Cleome gynandra L., Agkistrodon acutus (Gnther), Bungarus multicinctus Blyth, Elaphe moellendorffi (Boettger), Clematis maximowicziana Franch., Brassica alba (L.) Boiss., Perilla frutescens (L.) Britt., Phaseolus vulgaris L., Saccharum Sinensis Roxb., Salvia scapiformis Hance, Pinus bungeana Zucc., Pinus massoniana Lamb., Fraxinus malacophylla Hemsl., Sophora viciifolia Hance, Imperata cylindrica (L.) P. Beauv. Var. major (Nees) C. E. Hubb., Ginkgo biloba L., Acanthopanax trifoliatus (L.) Merr., Parnassia foliosa Hook. f. et Thoms. var. nummularia Nakai, P. wightiana wall., Buddleia asiatica Lour., Tilia omeiensis Fang, Chelidonium majus L., Chelidonium majus L. var. grandiflorum DC., P727, Coluber spinalis (Peters), Beesia calthaefolia (Maxim.) Ulbr., Quercus fabri Hance, Fragaria nilgerrensis Schlocht., Stephania cepharantha Hayata, Dioscorea panthaica Prain et Burkill, Pteroxygonum giraldii Dammer et Diels, Mallotus apelta (Lour.) Muell.-Arg., Lindera gambleana Allen, Populus alba L., Cynanchum bungei Decne., Cynanchum auriculatum Royle ex wight, C. wilfordi (Maxim.) Hemsl., Betula pendula Roth, Anas domestica L., Sesamum indicum DC., Liquidambar taiwaniana Hance, Prunus mume (Sieb.) Sieb. et Zucc., Smilax lunglingensis wang et Tang, Asystasiella neesiana (wall.) Lindau, Mussaenda parviflora Miq., Mussaenda divaricata Hutch., Elaeagnus viridus Serv. var. delavayi Lecte., Anser domestica Geese, Canarium album (Lour.) Raeusch., Setaria italica (L.) Beauv., Ampelopsis japonica (Thunb.) Mak., Fraxinus chinensis Roxb., Dictamnus dasycarpus Turcz., Dictamnus angustifolius G. Don, D. albus var. caucasicus, Bombyx mori L., Beauveria bassiana (Bals.) Vuill., Argyreia acuta Lour., Argyreia lilliflora C. Y. wu, Dioscorea hispida Dennst., Symplocos paniculata (Thunb. ) Miq., Rhodiola henryi (Diels) Fu, Viola arcuata Bl., Melaleuca leucadendra L., Equus caballus (L.), Serissa serissoides (DC.) Druce, S. foetida Comm., Heracleum rapula Franch., Solanum lyratum Thunb., Punica granatum L. var. albescens DC., Punica granatum L. var. multiplex Sw., Senecio nagensium C. B. Clarke, Elaeagnus oldhamii Maxim., Pulsatilla chinensis (Bge.) Reg., Gerbera delavayi Franch., Populus davidiana Dode, Amomum cardamomum L., Styrax dasyantha Perk., Gentiana algida Pall., G. algida Pall. var. przewalskii (Maxim.) Kusnez., Iris dichotoma Pall., Cleome gynandra L., Agkistrodon acutus (Gwnther), Fluggea virosa (willd.) Baill., Isodon sculponieatus (Vant.) Kudo, Paederia scandens (Lour.) Merr. var. tomentosa (Bl.) Hand.-Mazz., Sophora viciifolia Hance, Ginkgo biloba L., Aristolochia championii Merr. et Chun, Buddleia asiatica Lour., Chelidonium majus L., Gynura divaricata (L.) DC., Mallotus apelta (Lour.) Muell.-Arg., Nelumbo nucifera Gaertn., Chrysanthemum morifolium Ramat., Fraxinus chinensis Roxb., Rhinacanthus nasuta (L.) Kurz, Argyreia acuta Lour., Bellamya quadrata (Benson), Ajuga decumbens Thunb., Pulsatilla chinensis (Bge.) Reg., Populus davidiana Dode, Rhododendron mucronatum G. Don, Dracocephalum heterophyllum Benth., Agkistrodon acutus (Gwnther), Oldenlandia diffusa (willd.) Roxb., Oldenlandia corymbosa L., O. tenellifora (B1.) O. Ktze., O. pinifolia (wall.) K. Schum., Sophora glauca Lesch. var. albescens Rehd. et wils., Gynura divaricata (L.) DC., Cucumis melo L., Polygala japonica Houtt., Fissistigma oldhamii (Hemsl.) Merr, Polygonum caespitosum Bl., Ficus comata Hand.-Mazz., Ficus religiosa L., Benincasa hispida (Thunb.) Cogn., Debregeasiaedulis (Sieb. et Zucc.) wedd., Ilex chinensis Sims, Malva verticillata L., Abutilon theophrasti Medic., Malva verticillata L., Hepialus armoricanus Oberthwr, Cordyceps sinensis (Berk.) Sacc., Kalopanax septemlobus (Thunb.) Koidz., Aralia chinensis L., P769, Scrophularia ningpoensis Hemsl., S. grossheimi, S. nodosa, P771, P772, Lancea tibetica Hook. f. et Thoms., wahlenbergia marginata (Thunb.) A. DC., Caryopteris incana (Thunb.)Miq., Lancea thibetica Hook. f. et Thoms., Cypripedium margaritaceum Franch., Pinellia ternate (Thunb.) Breit., Pinellia pedatisecta Schott, Portulaca grandiflora Hook., Elsholtzia ciliata (Thunb.) Hyland, Lobelia chinensis Lour., Christia obcordata (Poir.) Bahn. f., Pteris semipinnata L., Scutellaria barbata D. Don, Perularia ussuriensis (Reg.) Schltr., P784, Passiflora wilsonii Hemsl., Pterospermum heterophyllum Hance, Semiliquidambar cathayensis H. Y. Chang, Dendropanax proteus (Champ.) Benth., Sassafras tsumu Hemsl., Artocarpus hypargyraea Hance, Alectoria asiatica Du Rietz, Deutzia ningpoensis Rehd, Glycyrrhiza pallidiflora Maxim., Ficus taiwanicola Maxim. var. angustifolia (Cheng) Migo, Ficus heteromorpha Hemsl., Crepis phoenix Dunn, Cudrania cochinchinensis (Lour.) Kudo et Masam., Sapindus delavayi (Franch.) Radlk., Vernonia andersonii Clarke, Pteracanthus alatus (wall. et Nees) Brem., Cassia alata L., Ceropegia pubescens wall. , Cynanchum hancockianum (Maxim.) Al. Iljinski, Satyrium nepalense D. Don, Passiflora cupiformis Mast., Polystichum tsus-simense (Hook.) J. Sm., Callicarpa rubella Lindl., Viburnum ichangensis Rehd., Chloranthus serratus (Thunb.) Roem. et Schult., Nanocnide japonica Bl.,
Quercus liaotungensis Koidz., Luffa cylindrica (L.) Roem., Luffa acutangula roxb., P792, Drymotaenium miyoshianum (Mak.) Mak., Euonymus bungeanus Maxim., Lindernia crustacea (L.) F. Mucll., Matricaria chamomilla L., Syzygium aromaticum (L.) Merr. et Perry, Cayratia carnosa Gagn., Arisaema purpureo-galeatum Engl., Halite, Viola yedoensis Mak., Viola japonica Langsd., Gueldenstaedtia multiflora Bge., Gueldenstaedtia pauciflora (Pall.) Fisch., Gentiana loureiri (D. Don) Griseb., Viola inconspicua Bl., V. patrinii DC., V. oxycentra Juz., Corydalis bungeana Turcz., Anemone flaccida Fr. Schmidt, Pachyrhizus erosus (L.) Urban, Burmannia coelestis D. Don, Melo coarctatus Motsch., Lycopus lucidus Turcz., P803, Melastoma dodecandrum Lour., Thymus serpyllum L., Thymus mongolicus Ronn., Heteropogon contortus (L.) Beauv., Sanguisorba officinalis L., Sanguisorba parviflora (Maxim.) Takeda, S. tenuifolia Fisch. et Link, S. grandiflora (Maxim.)Mak., S. longifolia Bertol., Parthenocissus tricuspidata (Sieb. et Zucc.) Planch., Astragalus bhotanensis Bak., Stephania delavayi Diels, Lithospermum zollingeri DC., Lithospermum arvense L., Viola diffusa Ging., Pachyrhizus erosus (L.) Urban, Ficus tikoua Bur., Ruellia drymophila (Deils) Hand-Mazz., Hypericum japonicum Thunb., Kadsura heteroclita (Roxb.) Craib, Lotus corniculatus L., Luzula capitata (Miq.) Miq., L. multiflora (Ehrh.) Lej., Polygala crotalarioides Buch.-Ham., Abacopteris penangiana (Hook.) Ching, Pratia begonifolia (wall.) Lindl., Kochia scoparia (L.) Schrad., Kochia scoparia (L.) Schrad. f. trichophila Schinz et Thell., K. sieversiana (Pall.) C. A. Mey., Chenopodium album L., Asplenium incisum Thunb., Selaginella moellendorfii Hieron., Viola philippica Cav. subsp. malesica w. Beck., Lycium chinense Mill., Myrmeleon micans Mac Lachlan, Drosera spathulata Labill., Urena lobata L., Viola collina Bess., Rubus xanthocarpus Bur. et Franch., Veronica serpyllifolia L., Cynanchum thesioides (Freyn) K. Schum., Marchantia polymorpha L., Melastoma dodecandrum Lour., Rehmannia glutinosa (Gaertn.)' Libosch., Viola grypoceras A. Gray, Munronia henryi Harms, Rehmannia glutinosa (Gaertn.) Libosch., Mollugo peritaphylla L., Euphorbia humifusa willd., Euphorbia pilulifera, Chamaerhodos erecta (L.) Bge., Raphanus sativus L., Gnaphalium adnatumwall. ex DC., StellariasaxatilisBuch.-Ham.Rubusiremaeus Focke, Ficus tikoua Bur., Kadsura heteroclita (Roxb.) Craib, Cotoneaster horizontalis Decne. var. perpusilla.Schneid., Ophiorrhiza succirubra King ex Hook. f., Musella lasiocarpa (Franch.) C. Y. wu, P831, Abies sutchuenensis (Franch.) Rehd. et wils., Celtis sinensis Pers., Colocasia esculenta (L.) Schott, Trillium tschonoskii Maxim., Trillium camtschaticum Pall., Achnatherum splendens (Trin.) Nevski, Miscanthus sinensis Anderss., Mirabilite, Hypericum patulum thunb., Dicranopteris dichotoma (Thunb.) Bernh., Euphrasia maximowiczii wettst., Sesamum indicum DC., Reineckea carnea Kunth, Lobaria pulmonaria (L.) Hoffm., Bauhinia variegata L., Leontopodium leontopdioides (wils.) Beauv., Cynanchum inamoenum (Maxim.) Loes., Rubus pinfaensis Levl. et Vant., Polygonatum cirrhifolium(wall.) Royle, Diospyros rhombifolia Hemsl., Callicarpa bodinieri Levl. var. giraldii (Rehd.) Rehd., Thladiantha verrucosa Cogn. ex Oliv., Medicago lupulina L. , Capparis spinosa L., Berchemia lineata (L.) DC., Ilex pernyi Franch., Acanthus ilicifolius L., Erodium stephanianum willd., Geranium wilfordii Maxim., Geranium nepalense Sweet, Geranium sibiricum L., Geranium carolinianum L., G. dahuricum DC., G. dahuricum DC. var. alpinum Bar. et Skv., Amaranthus caudatus L., Mucuna wangii Hu, Mucuna macrocarpa wall., Brucea javanica (L.) Merr., Sabia yunnanensis Franch., Epaltes australis Less., Bauhinia variegata L., Phryma leptostachya L., Citrullus vulgaris Schrad., P850, Mactra antiquata Spengler, Panax quinquefolium L., Passiflora caerulea L., Nasturtium officinale R. Br., Linum usitatissimum L., Oryza sativa L., Oroburea (L.) Pers., Chenopodium serotinum L., Polystichumdeltodon (Bak.) Diels, Cotoneaster acutifolius Turcz., Lilium brownii F. E. Browm var. colchesteri wils., Lilium pumilum DC., Lilium longiflorum Thunb., Lilium dahuricum Ker-Gawl., L. concolor Salisb., L. cernuum Kom. , L. concolor Salisb. var. buschianum Bak., L. distichum Nakai, L. davidii Duch. , L. martagon L. , Stemona japonica (Bl.) Miq., Stemona sessilifolia (Miq.) Franch. et Sav., Stemona tuberosa Lour., Stemona parviflora wright, S. vagula w. w. Smith., Asparagus filicinus Ham. ex D. Don, A. officinalis L., Turdus merula mandarinus Bonaparte, Ardisia crispa (Thunb.) A. DC., Aridisia brevicaulis Diels, Marsdenia longipesw. T. wang, Disporum sessile (Thunb.) D. Don var. flavens (Kitag.) Y. C. Tang, Disporum cantoniense (Lour.) Merr., Aletris lanuginosa Bur. et Franch., P864, P865, Lotus corniculatus L., Morinda parvifolia Bartl., Thesium chinense Turcz., Ardisia crispa (Thunb.) A. DC., Gymnotheca chinensis Decne., Neottianthe cucullata (L.) Schltr., Nerium indicum Mill., Cyclemys sp., Cyclemys trifasciata Bell, Polygonum perfoliatum L., Cynanchum officinale (Hemsl.) Tsiang et Zhang, Seseli seseloides (Fisch. et Mey. ex Turcz.)Hiroe, Ampelopsis aconitifolia Bge., Reinwardtia trigyna (Roxb.) Planch., Lycopodium complanatum L., Lycopodium casuarinoides Spring, Adiantum flabellulatum L., Jussiaea repens L., Embelia pauciflora var. blinii (Levl.) walker, Bauhinia championi (Benth.) Benth., Callicarpa longissima (Hemsl.) Merr., Cyrtomium caryotideum (wall.) Presl, Angelica sinensis (Oliv.) Diels, Angelica acutiloba (Sieb. et Zucc.) Kitag., Ligusticum glaucescens Franch., Levisticum officinale Koch, Angelica acutiloba var. sugiyama Hikino, Embelia parviflora wall., Pyrrosia clavata (Bak.) Ching, P880, Tulipaedulis Bak., Tulipailiensis Reg., Croton laevigatus Vahl, Stachys palustris L., Trema cannabina Lour., Sedum drymarioides Hance, Bullacta exarata (Philippi), Pellionia repens (Lour.) Merr., Strychnos ignatii Berg., Mallotus philippinensis (Lam.) Muell.-Arg., Celastrus angulata Maxim., Viburnum cylindricum Buch.-Ham. ex D. Don, Zebrina pendula Schnizl., Ceropegia christenseniana Hand.-Mazz., Rubus innominatus S. Moore var. Kuntzeanus (Hemsl.)Bailey, Rhodobryum roseum Limpr., Cicer arietinum L., Ranunculus chinensis Bge., Atylosia mollis (willd.) Benth., Sanguisorba filiformis (Hook. f.)Hand.-Mazz., Phyllostachys nigra (Lodd.) Munro var. henonis (Mitf.) Stapf ex Rendle, Isodon ternifolius (D. Don) Kudo, Fraxinus chinensis Roxb., Ligustrum lucidum Ait., Exicerus pela(Chavannes), Cinnamomum cassia Presl, Myristica fragrans Houtt., Cistanche salsa (C. A. Mey.) G. Beck, Cistanche ambigua (Bge.) G. Beck, Impatiens microcentra Hand.-Mazz., Cinnamomum cassia Presl, Myristica fragrans Houtt., Phyllostachys nigra(Lodd.)Munro var. henonis (Mitf.) Stapf ex Rendle, Phyllostachys sulphurea (Carr.) A. et C. Riv., Bambusicola thoraica thoraciac (Temm.), Labeo decorus Peters, P900, Bambusa textiles Mc-Clure, Shiraia bambusicola Henn., Xylocopa dissimilis (Lep.), P903, Tupistra ensifolia wang et Tang, Commelina diffusa Burm. f., Homalocladium platycladum (F. Muell.) Bailey, Murdannia divergens (C. B. Clarke) Brcckn.,Disporum cantoniense (Lour.) Merr., Pollia japonica Thunb., Commelina benghalensis L., Zanthoxylum planispinum Sieb. et Zucc., Conax Cannaeformis (Forst.) Schum., Tecomaria capensis (Thunb.) Spach, Streptopus simplex D. Don, Lingnania chungi McClure, Sinocalamus affinis (Rendle) McClure, Tupistra chinensis Bak., Rhizomys sinensis Gray, Lyctus brunneus Steph., Panax pseudo-ginseng wall. var. japonicus (C. A.Mey.) Hoo & Tseng, Panax pseudo-ginseng wall. var. bipinnatifidus (Seem.) Li, P. pseudo-ginseng wall. var. angustifolius (Burkill) Li, Anemone raddeana Reg., Lathyrus palustris L. var. linearifolius Ser., Lathyrus sativus, Seseli mairei wolff , Seseli yunnanense Franch., Zanthoxylum planispinum Sieb. et Zucc., Rhizomys sinensis Gray, Lyctus brunneus Steph., Claoxylon polot(Burm. f.) Merr., Erythrina arborescens Roxb., Callicarpa arborea Roxb., Cinnabar, Salvia cavaleriei Levl.,Ardisia crenata Sims, Aristolochia kaempferi willd., Damnacanthus indicus Gaertn. f., P915, Vernonia cinerea (L.) Less., Symplocos chinensis (Lour.) Druce, Physochlaina infundibularis Kuang, Impatiens chinensis L., Combretum alfredii Hance, Chrysosplenium sinicum Maxim., Corydalis yanhusuo w. T. wang, Corydalis ambigua Cham. Et Schlecht. var. amurensis Maxim., C. ambigua Cham. et Schlecht., C. remota Fisch. ex Maxim., Corydalis hamosa Migo, Corydalis remota Fisch var. lineariloba Maxim, Corydalis remota Fisch var. pectinata Kom, Ranunculus cantoniensis DC., Crotalaria assamica Benth., Pyrite, P925, wedelia wallichii Less., Taxus mairei (Lemee et Levl.) S. Y. Hu, Daemonorops draco Bl., Pleomele cambodiana (Pier. ex Gagn. ) Merr. et Chun, Schisandra henryi Clarke, Schisandra sphenanthera Rehd. et wils., Lespedeza buergeri Miq., Chenopodium hybridum L.,Ventilagoleiocarpa Benth., Sambucus adnata wall., Galinsoga parviflora Cav., Sesbania cannabina (Retz.) Pers., Helianthus annuus L., Pheropsophus jessoensis Mor., Buthus martensi Karsch, Androctonus australis, Dracocephalum integrifolium Bge., Rubus amphidasys Focke, Aeschymomene indica L., Actinostemma lobatum (Maxim.) Maxim., Albizzia julibrissin Durazz., Albizzia chinensis, Albizzia kalkora (Roxb. ) Prain, A. gummifera, Albizzia julibrissin Durazz. , Aeschynomene indica L., Polygala lateriflora Y. K. Yang et al., Cynanchum amplexicaule Hemsl., Polygonum polystachyum wall., Zanthoxylum multijugum Franch., Balanophora polyandra Griff., Clinopodium polycephalum (Van.) C. Y. wu et Hsuan, Dysosma auranticocaulis (Hand.-Mazz.) Hu, Melastoma polyanthum B1., Meconopsis horridula Hook.f. et Thoms., Vicia multicaulis Ledeb., Lithocarpus polystachyus Rehd., Alnus tinctoria Sarg., Anas platyrhynchos L., Artemisia anomala S. Moore, Siphonostegia chinensis, Benth., Artemisia selengensis Turcz., Lepisma saccharina L., Equisetum arvense L., Arctia caja L., Juncus effusus L. var. decipiens Buchen., Juncus leschenaultii Gay., J. setchuensis Buchen. var. effusoides Buchen., Ribes mandschuricum (Maxim.) Kom., Physalis peruviana L., Juncus effusus L. var. decipiens Buchen., Erigeron breviscapus (Vant.) Hand.-Mazz., Erigeron bonariensis, Cassia tora L., P951, Dryobalanopsaromatica Gaertn. f., Blumea balsamifera DC., P953, Aneurolepidium dasystachys (Trin.) Nevski, Pinna pectinata L., Hydrocotyle sibthorpioides Lam. var. batrachium (Hance) Hand.-Mazz., Hydrocotyle sibthorpioides Lam., Stephania hernandifolia walp., Campylotropis macrocarpa (Bge.) Rehd., Styrax benzoin Dryand., Styrax tonkinensis (Pier.) Craib, Styrax hypoglauca Perk., S. macrothyrsus Perk., S. subnivea Merr. et Chun, Aristolochia manshuriensis Kom., Aconitum coreanum (Levl.) Raipaics, Capra hircus L., Ovis aries L., Rumex japonicus Houtt., Rumex nepalensi Spr., Rumex confertus willd., Rumex japonicus Houtt., Rumex nepalensis Spr., Zanthoxylum dimorphophyllum Hemsl., Liparis japonica (Miq.) Maxim., Strophanthus divaricatus (Lour.) Hook. et Arn., Polygonatum verticillatum (L.) All., Polygonatum roseum (Ledeb.) Kunth, Lindernia angustifolia (Benth.) wettst., Morinda umbellata L., Osmanthus matsumuranus Hayata, Rumex japonicus Houtt., R. nepalensis Spr., Emilia sonchifolia (L.) DC., Laggera alata (Buch.-Ham.) Sch.-Bip., Buddleia officinalis Maxim., Strophanthus divaricantus (Lour.) Hook. et Arn., Carex lanceolata Boott, Viburnum utile Hemsl., Rhododendron molle (Bl.) G. Don, Passiflora altebilobata Hemsl., P973, P974, Aglaia odorata Lour., Oryza sativa L., P975, Eurya chinensis R. Br., Vaccinium sprengelii (G. Don) Sleumer, Erigeron canadensis L. , Allophylus viridis radlk. , Cyperus difformis L., Aristolochia mollissima Hance, Averrhoa carambola L., Actinolite, Actinolite asbestus, Actinolitum brevifibrum, Caragana franchetiana Kom., Platysternon megacephalum Gray, Botrychium ternatum (Thunb.)Sw., Cinnamomum burmannii (Nees) B1., Stephania tetrandra S. Moore, Aristolochia fangchi wu, Cocculus trilobus (Thunb.) DC., Aristolochia heterophylla Hemsl., Sinomenium acutum (Thunb.) Rehd. et wils., Menispermum dauricum DC., Aristolochia moupinensis Franch., A. kaempferi willd., Saposhnikovia divaricata (Turcz.) Schischk., Ligusticum brachylobum Franch., Seseli mairei wolff, Seseli yunnanense Franch., Seseli iliense (Reg. Et Sclmalh.) Lipsky, Saposhnikovia divaricala (Turcz.) Schischk., Anisomeles indica (L.) O. Ktze., Gynura bicolor DC., Microsorium dilatatum (Bedd.) Sledge, Syringa pinnatifolia Hemsl., Panax pseudo-ginseng wall. var. bipinnatifidus (Seem.) Li, Gnetumparvifolium (warb.) C. Y. Cheng, Styrax suberifolia Hook. et Arn. , Remusatia vivipara (lodd.) Schott., Monascus purpureus went, Ormosia hosiei Hemsl. et wils., Carthamus tinctorius L., Arisaema balansae Engl., Solanum septemlobum Bge., Polygonum suffultum Maxim., Desmodium szechuenense (Craib) Schindl., Orchis salina Turcz., Pyracantha fortuneaha (Maxim.) Li, Begonia fimbristipulata Hance, Begonia membranifera Chun et F. Chun, Carrierea calycina Franch., Iresine herbstii Hook. f., Kadsura longipedunculata Finet et Gagn., Tetrastigma obovatum(Laws.) Gagn., Leontice robustum (Maxim.) Diels, Centropus sinensis (Stephens), C. bengalensis (Gmelin), Murdannia malabaricum (L.) Brckn., Eupatorium heterophyllum DC., Umbilicaria hypococcina (Jatta) Llano, Gyrophora tornata Ach., Dermatocarpon miniatum (L.) Mann., Blumea lacera (Burm. f.) DC., Peristrophe roxburghiana (Schult.) Brem., Larix potaninii Batal., Alpinia galanga (L.) Swartz, Carthamus tinctorius L., Astragalus sinicus L., Hypericum ascyron L., Actinodaphne cupularis (Hemsl.) Gamble, Strobilanthes japonicus (Thunb.) Miq., Laportea macrostachya (Maxim.) Ohwi, Polygonum ciliinerve (Nakai) Ohwi, Alchornea trewioides (Benth.) Muell.-Arg., Salvia plectranthoides Griff., Anneslea fragrans wall., Dalbergia hancei Benth., Begonia laciniata Roxb., Huechys sanguinea De Geer., Rhus punjabensis Stew. var. sinica (Diels) Rehd. et wils., Smilax glabra Robx., S. glauco-china warb., Smilax mairei Levl., Rubus rufo-lanatus H. T. Chang, Ulmus pumila L. var. pilosa Rehd., Callicarpa rubella Lindl., Mallotus barbatus (wall.) Muell.-arg., Rhodiola sacra (Prain ex Hamet) Fu, Machilus thunbergii Sieb. et Zucc., Aralia echinocaulis Hand.-Mazz., Cyathocline purpurea (Ham.) O. Ktze., Chamaenerion angustifolium (L.) Scop., Polygonum flaccidum Meissn., Polygonum blumei Meissn., Persicaria longiseta, Desmodium szechuenense (Craib) Schindl., Oxyria sinensis Hemsl., Hydrocotyle nepalensis Hook., Trifolium pratense L., Eupatorium heterophyllum DC., Alternanthera versicolor Reg., Begonia crassirostris Irmsch., Blumea mollis (D. Don) Merr., Viburnum ovatifolium Rehd., Desmodium gangeticum (L.) DC., Rhododendron arboreum Smith f. roseum Sweet, R. campylocarpum Hook. f. , Clematis brevicaudata DC., Elaeagnus henryi warb., Swertia erythrosticta Maxim., Ipomoea batatas Lam., Ilex sp., Illicium lanceolatum A. C. Smith, Ecdysanthera rosea Hook. et Arn., Rubus mallodes Focke, Begonia sinensis A. DC., Castanopsis hickelii A. Camus, Erigeron elongatus Ledeb., Alstonia yunnanensis Diels, Acanthopanax giraldii Harms, Ardisia mamillata Hance, Meconopsis punicea Maxim., Caragana rosea Turcz., Chamaenerion angustifolium (L.) Scop., Veronica ciliata Fisch., Agate, Ustilago nuda (Jens.) Rostr., Ustilago hordei(Pers.)Lagerh., Hordeum dislichon, Claviceps purpurea (Fr.) Tulasne, Secale cereale L., Claviceps microcephala Tulasne, C. purpurea (Fr.)Tulasne, Claviceps microcephala Ce-3, Bulbophyllum nconspicuum Maxim., Ophiopogon japonicus Ker-Gawl., Ophiopogon intermedius D. Don, Liriope spicata Lour., L. minor (Maxim.) Mak., L. kansuensis (Batal.) C. H. wright, Ophiopogon japonicus Ker-Gawl. var. genuinus Maxim., Silene conoidea L., Boehmeria gracilis C. H. wright, Dalbergia mimosoides Franch., Mappianthus iodoides Hand.-Mazz., Polygala tenuifolia willd., Indigofera neopolygaloides Hu, Phlomis tuberosa L., Quercus liaotungensis Koidz., Mallotus repandus (willd.) Muell.-Arg., Eucommia ulmoides Oliv., Cuculus poliocephalus Latham, Asarum forbesii Maxim., Juniperus rigida Sieb. et Zucc., Maesa japonica (Thunb.) Moritzi, Rhododendron simsii Planch., Cunninghamia lanceolata (Lamb.)Hook., Hippuris vulgaris L., Mangifera indica L., Myrica rubra Sieb. et Zucc., weigela japonica Thunb., Myrica esculenta Buch.-Ham., Myrica rubra Sieb. et Zucc., Glycine max (L.) Merr., Campylotropis delavayi (Franch.)Schindl., Iris sanguinea Hornem., Litsea cubeba (Lour.) Pers., L. pungens Hemsl., L. euosma w. w. Smith, Amomum cardamomum L., Peperomia reflexa (L. f.) A. Dietr., P1045~1046, Helicia erratica Hook. f., Papaver rhoeas L., Polycarpaea corymbosa (L.) Lam., Crossostephium chinense (L.) Mak. ex Cham. et Schltr., Daphne genkwa Sieb. et Zucc., wikstroemia chamaedaphne Meissn., Ulmus macrocarpa Hance, Brassica rapa L., Brassica campestris L. var. oleifera DC., Cymbopogon distans (Nees) A. Camus, Sonchus brachyotus DC., Amaranthus mangostanus L., Percocyprispingi (Tchang), Polemonium liniflorum V. Vassil., Polemonium coeruleum L., Zanthoxylum bungeanum Maxim., Zanthoxylum schinifolium Sieb. et Zucc., Zanthoxylum simulans Hance, Z. planispinum Sieb. et Zucc., Z. avicennac (Lam.) DC., Z. simulans Hance var. podocarpum (Hemsl.) Huang, Z. piperitum DC., Sorbus pohuashanensis (Hance) Hedl., Halenia corniculata (L.) Cornaz., Clematis pseudopogonandra Finet et Gagn., Malus asiatica Nakai, Trigonella ruthenica L., Paederus densipennis Bernh., Lysimachia lobelioides wall., Cissus discolor B1., Zanthoxylum bungeanum Maxim., Zanthoxylum schinifolium Sieb. et Zucc., Begonia cathayana Hemsl., Ophicalcite, Pottsialaxiflora (B1.) O. Ktze., Asarum maximum Hemsl., Polygonum runcinatum Buch.-Ham., P. runcinatum Buch.-Ham. var. sinense Hemsl., Dryopteris laeta (Kom.) C. Chr., Barleria lupulina Lindl., Barleria prionitis L. , Munronia sinica Diels, Oenanthe javanica (Bl.) DC., Brassica juncea (L.) Czern. et Coss., Atractylodes lancea (Thunb.) DC., Atractylodes chinensis Koidz., Atractylodes japonica Koidz. ex Kitam., A. chinensis Koidz. var. simplicifolia Kitag., A. chinensis var. quinqueloba, A. chinensis var. liaotungensis, Xanthium sibiricum Patr. ex widd., Indigofera hancockii Craib, Saxiglossum angustissimum (Gies.) Ching, Diploclisia glaucescens (Bl.) Diels, Euryale ferox Salisb., Phragmites communis Trin., Aloe vera L., Aloe ferox Mill., Aloe vera L. var. chinensis (Haw.) Berger, Piper boehmeriaefolium wall., Arundo donax L., Ruellia repens L., Musa basjoo Sieb. et Zucc., Caesalpinia sappan L., Perilla frutescens (L.)Britt. Var. crispa (Thunb.) Hand.-Mazz., P. frutescens (L.) Britt. Var. acuta (Thunb.) Kudo, P. frutescens (L.) Britt., Indiogofera carlesii Carib, Liquidambar orientalis Mill., Malva neglecta wallr., Blumea laciniata (Roxb.) DC., Ardisia gigantifolia Stapf, Viola acuminata Ledeb., Tetrastigma obtectum (wall.) Planch., Salvia substolonifera Stib., Pellionia radicans (sieb. et Zucc.) wedd., Alnus japonica Sieb. et Zucc., Thladiantha dubia Bge., SyzygiumbuxifoliumHook. et Arn., Phaseolus calcaratus Roxb., Phaseolus angularis wight, Halloysite, Geranium strictipes R. Kunth, Paeonia lactiflora Pall., Paeoniaobovata Maxim.,Paeonia veitchii Lynch, Paeonia anomala L., P. mairei Levl.,P. obovata Maxim.var.willmottiae (Stapf) Stern, P. hybrida Pall., Pyracantha fortuneana (Maxim.) Li, Saccharum sinensis Roxb., Poria cocos (Schw.) wolf, Polygonum runcinatum Buch.-Ham., Polygonum runcinatum Buch.-Ham. var. sinense Hemsl., Vespa simillima Smith., Dinodon rufozonatum (Cantor), Elatostema umbellatum Bl. Var. majus Maxim., Calystegia soldanella R. Br., Prunus armeniaca L., P. armeniaca L. var. ansu Maxim., Prunus sibirica L., P. mandshurica Koehne, Pimpinella candolleana wight et Arn., Pimpinella anisum, Prunus salicina Lindl., Maesa indica wall., Polygonum amphibium L., Crepis elongata babc., Rhodiola yunnanensis (Franch.) Fu, Delphinium anthriscifolium Hance, Sedum verticillatum L., Pedicularis rex C. B. Clarke ex Maxim., Euonymus fortunei (Turcz.) Hand.-Mazz., Hibiscus rosa-sinensis L., Amelanchier sinica(Schneid.)Chun, Amelanchier asiatica Engl., Cyperus michelianus (L.) Link, Corydalis suaveolens Hance, Heterocodon brevipes (Hemsl.) Hand.-Mazz. et Nannf., Jasminum amplexicaule Buch.-Ham., Oxalis stricta L., Primula vittata Bur. et Franch., P. sikkimensis Hook., Forsythia suspense (Thunb.) Vahl, Forsythia koreana, F. intermedia, Cercidiphyllum japonicum Sieb. et Zucc. var. sinense Rehd. et wils., P1114, wedelia prostrata (Hook. et Arn.) Hemsl., w. prostrata (Hook. et Arn.) Hemsl. var. robusta Mak., Salomonia cantoniensis Lour., Kadsura oblongifolia Merr:, Spiraea japonica L. f. var. acuminata Franch., Evodia rutaecarpa (Juss.) Benth., Evodia rutaecarpa (Juss.) Benth. var. officinalis (Dode) Huang, E. rutaecarpa (Juss.) Benth. var. bodinieri (Dode) Huang, Evodia xanthoxyloides F. Muell., E. xanthoxyloides, Baeckea frutescens L, Ilex asprella (Hook. et Arn.) Champ. ex Benth., Apium graveolens L. var. dulce DC., Salix matsudana Koidz., Tropaeolum majus L., Zephyranthes grandiflora Lindl., Ostrea rivularis Gould, Ostrea gigas Thunb., Ostrea talienwhanensis Crosse, Artemisia japonica Thunb., A. japonica Thunb. f. resedifolia Takeda, Paeonia suffruticosa Andr., Paeonia suffruticosa Andr. var. spontanea Rehd., P. papaveracea Andr., P. lutea Franch., P. delavayi Franch., P. potanini Kom., P. szechuanica Fang, P. thalictrumifolia C. Ho et S. Y. Chen, P. lutea Franch., P. yunnanensis Fang, Paeonia emodi, P. officinalis, Clematis heracleifolia DC., Vitex negundo L. var. cannabifolia (Sieb. et Zucc.) Hand.-Mazz., Ostrea rivularis Gould, Artemisia japonica Thunb., Rattus norvegicus Berkenhout, Laportea sinensis C. H. wright, Canis familiaris L., P1134, Dicranostigma leptopodum(Maxim.)Fedde, Bredia amoena Diels, Polygonum multiflorum Thunb., Cynanchum bungei Decne., C. auriculatum Royle ex wight, Lycopodium obscurum L., L. cernnum L., Tixospora sinensis(Lour.)Merr., Bretschneidera sinensis Hemsl., Polygonum coriaceum Samuels., Lycianthes lysimachioides (wall.) Bitt., Citrusmedica L. var. sarcodactylis (Noot.) Swingle, Sedumlineare Thunb., Briggsia chingii(Merr.)Chun, Sedum multicaule wall., Aquilaria agallocha Roxb., A. sinensis (Lour.) Gilg, Kalanchoe laciniata (L.) DC., Gleditsia sinensis Lam., Salix wallichiana Anderss., Gleditsia horrida Mak. , Mimosa pudica L., Oryza sativa L., Setaria italica (L.) Beauv., Circaea quadrisulcata (Maxim.) Franch. et Sav., Eriocaulon buergerianum Koern., Eriocaulon sieboldtianum Sieb. et Zucc. ex Stend., E. nudicuspe Maxim., E. sexangulare L., E. australe R. Br., Kadsura coccinea (Lem.) A. C. Smith, Zephyranthes candida Herb., Triplostegia glandulifera wall., Ligularia laesicotal Kitam., L. tsangchanensis (Franch.) Hand.-Mazz., Coelogyne occultata Hook. f., Incarvillea sinensis Lam., Incarvillea younghusbandii Sprague, Hypecoum erectum L., Chinemys reevesii (Gray), Rhododendron mucronulatum Turcz., Jasminum nudiflorum Lindl., Magnolia liliflora Desr.,. Magnolia denudata Desr,., Magnolia fargesii Cheng, M. campbellii Hook. f. et Thoms., Rubus sachalinensis Levl.,Viola dissecta Ledeb., Machilus yunnanensis Lec. var. duclouxii Lec., Rhamnus globosus Bge., Saruma henryi Oliv., Abies delavayi Franch., Viburnum foetidum wall., Viola serpens wall., Pugionium cornutum (L.) Gaertn., Elaeagnus angustifolia L., Salix cheilophila Schneid., Ammopiptanthus mongolicus (Maxim.) Cheng f., Astragalus complanatus R. Br., Astragalus chinensis L., Astragalus adsurgens Pall., Calligonum mongolicum Turcz., Ferula borealis Kuan, Oxytropis psammocharis Hance, Acronychia pedunculata (L.) Miq., Artemisia halodendron Turcz., Echinops gmelinii Turcz., Oldenlandia capitellata (wall.) O. Ktze., Ferula borealis Kuan, Inula salsoloides (Trucz.)Ostenf., Commiphora myrrha Engl., Balsamodendron ehrenbergianum Berg., Quercus infectoria Olivier, Cynips gallae-tinctoriae Olivier, Aquilaria agallocha Roxb., Aquilaria sinensis (Lour.) Gilg, P1172, Notopterygium incisum Ting, Notopterygium forbesii Boiss., Notopterygium franchetii Boiss., Batrachuperus pinchonii (David), Terminalia chebula Retz., Terminalia chebula Retz. var. gangetica Roxb., Limonium gmelinii (willd.) O. Ktze., Psoralea corylifolia L., Nothopanax delavayi(Franch.)Harms, Channa asiaticus (L.), Ganoderma j aponicum (Fr.) Lloyd, Ganoderma lucidum (Leyss. ex Fr.) Karst., Meliosma cuneifolia Franch., Viverra zibetha L., Viverricula indica Desmarest, Swainsonia salsula Taub., Pithecellobium lucidum Benth., Pithecellobium saman, Pithecellobine, Diospyros lotus L., Sambucus javanica Reinw., Equus asinus L., Ferula assafoetida L.,Ferula caspica Marsh.-Bieb., Ferula conocaula Eug., Fomes officinalis (Vill. et Fr.) Ames, Alyxia sinensis Champ. ex Benth., Trachyspermum ammi (L.) Sprague, Heteropappus altaicus (willd.) Novopokr., P1189, P1190, Lagenaria siceraria (Molina) Standl. var. depressa Ser., L. siceraria (Molina) Standl. var. gourda Ser., Aconitum carmichaeli Debx., Trigonotis peduncularis (Trev.) Benth., Millingtonia hortensis L. f., Lonicera japonica Thunb., Gallus gallus domesticus Brisson, Collybia albuminosa (Berk.) Petch, Rhodotypos scandens (Thunb.) Mak., Jurinea mongolica Maxim., Randia sinensis (Lour.) Roem. et Schult., Calathodes oxycarpa Sprague, Viburnum setigerum Hance, Incarvillea delavayi Bur. et Franch., Polygonum amplexicaule D. Don var. sinense Forb. et Hemsl., Prunus simonii Carr., Spatholobus suberectus Dunn, Mucuna birdwoodiana Tutcher, Millettia dielsiana Harms, Millettia nitida Benth., Sargentodoxa cuneata (Oliv.) Rehd. et wils., Mucuna sempervirens Hemsl., Millettia reticulata Benth., Chrysopogon aciculatus (Retz.) Trin., Elsholtzia blanda (Benth.) Benth., Habenaria delavayi Finet, Habenaria densa wall., Parnassia wightiana wall., Viburnum sargentii Koehne, Abrus fruticulosus wall. ex wight et Arn., Groton crassifolius Geisel., Dipelta ventricosa Hemsl., Celosia cristata L., Potentilla bifurca L., Gallus gallus domesticus Brisson, Paederia scandens (Lour.) Merr., Dioscorea cirrhosa Lour., Tripterygium wilfordii Hook. f., Boehmeria nivea (L.) Gaud., Plumbagella micrantha (Ledeb.) Spach, Kummerowia striata (Thunb.) Schindl., Kummerowia stipulacea (Maxim.)Mak.,Oxytropismyriophylla (Pall.) DC.,Berberischingii Cheng, Plumeria rubra L. var. acutifolia (Poir. ) Bailey, Codonopsis convolvulacea Kurz, Cornus capitata wall., Desmoscochinchinensis Lour., Stellaria neglecta weihe, Euphorbia esula L., Viburnum sargentii Koehne, Pogostemon glaber Benth., Paederia scandens (Lour.) Merr., Epipactis papillosa Franch., Gendarussa vulgaris Nees, Psychotria siamica (Craib) Hutch., Equus asinus L., Crepis turczaninowii C. A. Mey., Dendrobium moniliforme (L.) Sw., Rosa rugosa Thunb., Citrus tangerina Hort. et Tanaka, C. erythrosa Tanaka, Citrus sinensis (L.) Osbeck, C. wilsonii Tanaka, C. chachiensis Hort., Mylopharyngodon piceus (Richardson), Aster faberi Franch., Celosia argentea L., Rana nigromaculata Hallowell, Rana plancyi Lataste, Allium sativum L., Artemisia apiacea Hance, Artemisia annua L., Artemisia capillaris Thunb., A. scoparia waldst. et Kitaib., Artemisia japonica Thunb., Avena nuda L., Clinacanthus nutans (Burm. f.) Lindau, Baphicacanthus cusia (Nees) Brem., Indigofera tinctoria L., Isatis tinctoria L., I. indigotica Fort., Polygonum tinctorium Ait., Nervilia fordii (Hance) Schltr., Aristolochia debilis Sieb. et Zucc., Aristolochia contorta Bge., Aristolochia kaempferi willd., A. chuii C. Y. wu, Vladimiria berardioides (Franch.) Ling, Vladimiria forrestii (Diels) Ling, Vladimiria edulis (Franch.) Ling, Sinomenium acutum (Thunb.) Rehd. et wils., Diploclisia chinensis Merr., Sabia japonica Maxim., Cocculus trilobus(Thunb.)DC., Paederia scandens (Lour.) Merr., Hedera nepalensis K. Koch var. sinensis(Tobl.)Rehd., Swertia vacillans (Hance) Maxim., Swertia pulchella (D. Don)Buch.-Ham., S. nervosa wall., Lycopodium hamiltonii Spr., Hypoestes purpurea (L.) Soland., Rhaphidophora hongkongensis Schott, Trimeresurus stejnegeri Schmidt, Cynanchum otophyllum Schneid., Quercus variabilis Bl., Quercus semicarpifolia Smith, Prinsepia utilis Royle, Mylopharyngodon piceus (Richardson), Aristolochia calcicolaC. Y. wu, Desmodiumcaudatun (Thunb. ) DC., Lyttacaraganae Pallas, Celosia argentea L., Rana nigromaculata Hallowell, R. plancyi Lataste, Artemisia apiacea Hance, A. annua L., Setaria italica (L.) Beauv., Cocculus trilobus (Thunb.) DC., Lysionotus serratus D. Don, Ainsliaea rubrinervis Chang, Tripterospermum japonicum (Sieb. et Zucc.) Maxim., Cordia dichotoma Forst. F., P1244, Desmodium caudatum (Thunb.)DC., Rhus potanini Maxim., Rhus chinensis Mill., P1244, Aster lasiocladus Hayata, Pileostegia viburnoides Hook. f. et Thoms., Saxifraga przewalskii Engl., Gymnema sylvestre (Retz.) Schult., Malus asiatica Nakai, Rhus succedanea L., Lespedeza davurica (Laxm.) Schindl., Cyathula prostrata (L.) B1., Eriobotrya japonica (Thunb.) Lindl., Xylanche himalaica G. Beck, Isatis tinctoria L., Isatis indigotica Fort., Baphicacanthus cusia (Nees). Brem., Pinus tabulaeformis Carr., Pinus massoniana Lamb., Pinus yunnanensis Franch., Usnea longissima Ach., Usnea diffracta Vain., Phtheiros permum japonicum(Thunb.)Kanitz, Sciurus vulgaris L., Armillaria matsutake Ito et Imai, Picris hieracioides L. subsp. j aponica Krylov, Pterocarya stenoptera DC., Dendropanax chevalieri(Vig.)Merr., Liquidambar taiwaniana Hance, Viscum articulatum Burm. f., Broussonetia kazinoki Sieb. et Zucc., Lindera umbellata Thunb., Ribes alpestre wall. ex Decne., Morina delavayi Franch., Trapa incisa Sieb. et Zucc. var quadricaudata Gluck, Rubus hirsutus Thunb., Rosa roxburghii Tratt. F. normalis Rehd. et wils., Hemiptelea davidii (Hance) Planch, Alhagi pseudalhagi Desv., Chenopodium aristatum L., Oplopanax elatus Nakai, Acanthopanax trifoliatus (L. ) Merr., Solanum khasianum C.B. Clarke, Rosa omeiensis Rolfe, Aralia elata (Miq.) Seem., Bambusa sinospinosa McClure, Salsola ruthenica Iljin, Rosa davurica Pall., Erythrina variegata L. var. orientalis (L.) Merr., Rosa graciliflora Rehd. et wils., Smilax ferox wall. ex Kunth, Berberis soulieana Schneid., Mahonia gracilipes (Oliv.) Fedde, Mahonia ganpinensis (Levl.) Fedde, Mahonia fortunei (Lindl.) Fedde, Berberis anhweiensis Ahrendt, Cirsium belingschanicum Petr., Erinaceus europaeus L., Robinia pseudoacacia L., Tribulusterrestris L., Smilax nana wang, Euonymus wilsonii sprague, Caesalpinia crista L., Celastrus flagellaris Rupr., Kalopanax septemlobus (Thunb.) Koidz., Morina coulteriana Royle, Eragrostis pilosa (L.) Beauv., Scutellaria orthocalyx Hand.-Mazz., Jasminumsambac (L.) Ait., Lactuca taiwaniana Maxim., Momordica charantia L., Lactuca versicolor (Fisch.) Sch.-Bip., Cirsium chinense Gardn. et Champ., Solanum dulcamara L., Sophoraflavescens Ait., Vallisneria spiralis L., Synedrella nodiflora (L.) Gaertn., Sonchus oleraceus L., Centaurea picris Pall., Physalis pubescens L., Ilex cornuta Lindl., Ilex latifolia Thunb., Ligustrum pedunculare Rehd., L. japonicum Thunb. var. pubescens Koidz., Sophora alopecuroides L., Caesalpinia minax Hance, Corydalis bungeana Turcz., Elephantopus scaber L., Elephantopus tomentosus L., Pleioblastus amarus (Keng) Keng f., SophoraalopecuroidesL., Astragalus melilotoides Pall., Picrasma quassioides (D. Don) Benn., FagopyrumtataricumGaertn., Ligustrum japonicum thunb. var. pubescens Koidz., Ixeris denticulata (Houtt.) Stebb., Lagenaria siceraria (Molina) Standl. var. gourda Ser. , Pueraria omeiensis wang et Tang, Melia azedarach L., Melia toosendan Sieb. et Zucc., Ixeris sonchifolia (Bge.) Hance, I. sonchifolia (Bge.) Hance var. serotina (Maxim.) Kitag., Millettia pachycarpa Benth., Scorzonera divaricata turcz., Loranthus chinensis DC., Brassica caulorapa Pasq., Marsilea quadrifolia L., Malus pumila Mill., Medicago sativa L., Medicago hispida Gaertn. , Medicago hispida Gaertn. Var. denticulata, M. hispida Gaertn. var. denticulata, Clematis glauva willd., Abutilon theophrasti Medic., Rosa bracteato wendl., Boehmeria nivea (L.) Gaud., Asarum insigne Diels, Solanum melongena L., P1311, Castanea seguinii Dode, Roegneria serotina Keng, Hierochlo odoranta (L.) Beauv., Castanea henryi (Skan) Rehd. et wils., Drosera peltata Smith var. lunata (Buch.-Ham.) C. B. Clarke, Monochoria korsakowii Reg. Et Maack, Curcuma aromatica Salisb., Curcuma zedoaria (Berg.) Rosc., Prunus japonica Thunb., Prunus humilis Bge., Prunus japonica Thunb. var. nakaii (Levl.) Rehd., Tulipa gesneriana L., Delphinium monanthum Hand.-Mazz., Pittosporum oligocarpum Hayata, Sida szechuensis Matsuda, Lagerstroemia subcostata Koehne, Drymoglossum piloselloides (L.) Presl, Clematis clarkeana Levl. et Van't., Veratrum taliense Loes. F., Ainsliaea lancifolia Franch., Senecio argunensis Turcz., Veronicastrum sibirica (L.) Pennell, Veronicastrum sibirica (L.) Pennell. var. japonicum, Oxytropis chiliophylla Royle, Actinidia arguta (Sieb. et Zucc.) Planch., Atriplex sibirica L., Thalictrum ramosum Boivin, Arsenopyrite, Iris tectorum Maxim., Milvus korschun lineatus (Gray), Odontites serotina Reich., Eritrichium rupestre (Pall.) Bge., Lindernia ciliata (Colsm.) Pennell, Schizophragma integrifolium Oliv. f. denticulatum (Rehd.) Chun, Polygonum cuspidatum Sieb. et Zucc., Damnacanthus indicus Gaertn. f., Panthera tigris L., Anectochilus taiwanensis Hayata, Chrysosplenium macrophyllum Oliv., Saxifraga stolonifera (L.) Meerb., Sansevieria trifasciata Prain, Sansevieria trifasciata Prain var. laurentii N. E. Br., Uraria crinita Desv, Delphinium tatsienense Franch. aff., Anemone rivularis Buch.-Ham., Corallodiscus cordatulus (Craib) B.L.Burtt, Nephrolepis cordifolia (L.) Presl, Veronica persica Poir., Ainsliaea spicata Vant. var. obovata (Franch. ) C. Y. wu, Pandanus furcatus Roxb., Patrinia villosa Juss., Patrinia scabiosaefolia Fisch., Sonchus brachyotus DC., Thlaspi arvense L., Changium smyrnioides wolff, Moghania fluminalis (C.B.Clarke) Li, Rodgersia sambucifolia Hemsl., Rodgersia pinnata Franch., Sedum leucocarpum Franch., Sinocrassulaindica (Decne.) Berger, Thunia alba (Lindl.) Reichb f., Zanthoxylum acanthopodium DC. var. villosum Huang, Oberonia myosurus (Forst.) Lindl., Artemisia brachyloba Franch., Codonopsis purpurea wall., Melodinus henryi Craib, Dichocarpum dalzielii (Drumm. et Hutch.) w. T. wang et Hsiao, Bergenia purpurascens (Hook.f.et Thoms.) Engl., Bergenia crassifolia (L.) Fritsch, Boea crassifolia Hemsl.Thalictrum ichangense Lecoy. ex Oliv., Rhodobryum giganteum (Hook.) Par., Dracocephalum rupestre Hance, Patrinia rupestris Juss., Ophiorrhiza japonica B1. var. leiocalyx Hand.-Mazz., Lysionotus carnosa Hemsl., Briggsia fritschii (Levl. et Vant.) Craib, Bergenia purpurascens (Hook. f. et Thoms.) Engl., Corydalis thalictrifolia Franch., Boenninghausenia albiflora (Hook.) Meissn., B. sessilicarpa Levl., Boeninghausenia albiflora Meissn. var. japonica S. Suzuki, Berneuxia thibetica Decne., Vaccinium delavayi Franch., Campanula colorata wall., Millettia kweichowensis Hu, Impatiens davidii Franch., Epiphyllum oxypetalum Haw., Laminaria japonica Aresch., Ecklonia kurome Okam., Undaria pinnatifida (Harv.) Sur., Aconitum vilmorinianum Kom., Millettia reticulata Benth., Berberis kunmingensis C. Y. wu , Berberis pruinosa Franch., Ocimumbasilicum L., Apocynum venetum L., Momordica grosvenori Swingle, Hemsleya macrosperma C. Y. wu, Hemsleya amabilis Diels, Hemsleya chinensis Cogn., Thladiantha hookeri C. B. Clarke, Crinum asiaticum L.var. sinicum Bak., Podocarpus macrophyllus (Thunb.) D. Don var. maki (Sieb.) Endl., P. macrophyllus (Thunb.) D. Don, Podocarpus forrestii Craib et w. w. Smith, Bulbophyllum odoratissimum (J. E. Smith) Lindl., Bos grunniens L., Thermopsis lanceolata R. Br., Chlorophytum capense (L.) Ktze., Neillia sinensis Oliv., Veronicastrum axillare (Sieb. et Zucc.) Yamazaki subsp. venosum (Hemsl.) Hong, Veronicastrum axillare (Sieb. et Zucc.) Yamazaki subsp. latifolium (Hemsl.) Hong, Veronicastrum axillare (Sieb. et Zucc.) Yamazaki subsp. stenostachyum (Hemsl.) Hong, Lindera umbellate Thunb., Luisia morsei Rolfe, waltheria americana L., Lespedeza davidii Franch., Cucubalus baccifer L. var. japonicus Miq., Hordeum vulgare L. var. nudum Hook. f., Anemarrhena asphodeloides Bge., Excoecaria acerifolia F. Didr., Potentilla chinensis Ser., Amaranthus spinosus L., Lasia spinosa (L.) Thw., Euonymus oxyphylla Miq., Malus halliana Koehne, Arabis pendula L. , Quisqualis indica L. , Biota orientalis (L.) Endl., Cupressus torulosa D. Don, Sabina recurva (Buch.-Ham.) Antoine, Eupatorium fortunei Turcz., Eupatorium cannabinum L., Begonia cavalerei Levl.,Rhaphidophora decursiva (Roxb.) Schott, Campylotropis trigonoclada (Franch.) Schindl., Boswellia carterii Birdw., Boswellia bhaw-dajiana Birdw., B. neglecta M. Moore, P1381, Anaphalis lactea Maxim., Anaphalis lacteal Maxim. f. rosa Lang, Viola philippica Cav., Carassius auratus (L.), Paraphlomis rugosa (Benth.) Prain var. coronata (Van't.) C. Y. wu, Native gold, Fortunella margarita (Lour.) Swingle, Fortunella crassifolia Swingle, Fortunella japonica (Thunb.) Swingle, Callicarpa kwangtungensis Chun, Stephania sinica Diels, Aconitum taiPeicum Hand.-Mazz., Polygala telephioides willd., Astilbe myriantha Diels, Lysimachia grammica Hance, Quamoclit pennata (Lam. ) Boj., Craibiodendron yunnanense w. w. Smith, Dischidia chinensis Champ. ex Benth., Dischidia australis Tsiang, Alectoria virens Tayl., Melica scabrosa Trin., Hypericum chinense L., Trichosanthes obtusiloba C . Y. wu, Asarum longepedunculatum O. C. Schmidt, Coleus pumilus Blanco, Smilax scobinicaulis c. H. wright, Smilax glauco-china warb., Ampelopsis delavayana (Franch.) Planch., Smilax bockii warb., Lotus tenuis Kitag., Pteris dactylina Hook., Cinchona succirubra Pav., Cinchona ledgeriana Moens., C. calisaya wedd., C. officinalisL., Triumfetta pilosa Roth, Kyllinga triceps Rottb., Bungarus fasciatus (Schneider), Tinospora capillipes gagn., Tinospora imbricata S. Y. Hu, Potentilla reptans L. var. sericophylla Franch., Inula britannica L. var. chinensis (Rupr.) Reg., I. linariaefolia Turcz., I. britannica L., Cladonia fallax Abbayes, Percocypris grahami (Regan), Antenoron filiforme (Thunb.) Roberty et Vautier, Antenoron neofiliforme (Nakai) Hara, Carpesium divaricatum Sieb. et Zucc., Phymatopsis griffithiana (Hook.) J. Sm., Calendula arvensis L., Calendula officinalis L., Trollius chinensis Bge., Trollius asiaticus L., Glechoma longituba (Nakai) Kupr., Lysimachia christinae Hance, Psammosilene tunicoides w. C. wu et C. Y. wu, Clematis huchouensis Tamura, Caragana sinica (Buc'hoz) Rehd., L. dasystyla Rehd., L. similis Hemsl., L. fuchsioides Hemsl., L. henryi Hemsl., L. lanceolata wall., Hyla chinensis Gnther, Hyla regilla, wikstroemia nutans Champ., Vermiculite, Rosa laevigata Michx., Rosa bella Rehd. et wils. , R. odorata Sweet var. gigantea Rehd. et wils. , R. macrophylla Lindl., Ainsliaea fragrans Champ., Clematis loureiriana DC. var. subpeltata (wall.) Hand.-Mazz., Euonymus yunnanensis Franch., Parameria laevigata (Juss.) Moldenke, Pittosporum glabratum Lindl. var. neriifolium Rehd. et wils., Smilax glauco-china warb., Amphicome arguta (Royle) Lindl., Bidenspilosa L., Bidensbiternata (Lour.) Merr. et Sherff, Limonium aureum (L.) Hill, Paliurus hemsleyanus Rehd., Lonicera japonica Thunb., Agave americana L. var. marginata Hort., Agave americana L., Agave schottii, Rhododendron przewalskii Maxim., Chrysosplenium grayanum Maxim., Macrothelypteris oligophlebia (Bak.) Ching, Macrothelypteris oligophlebia (Bak. ) Ching var. elegans (Koidz. ) Ching, Phegopteris decursive-pinnata Fe, Vulpes vulpes L., Uraria lagopodioides (L.) Desv., Canis familiaris L., Cibotium baromets (L.) J. Sm., woodwardia japonica (L. f.) Sm., Huolirion montana (Dammer) wang et Tang, Stizolobium cochinchinensis (Lour.) Tang et wang, Machilus bracteata Lecomte, Senecio integrifolius (L.) Clairvill var. fauriei (Levl. et Vant. ) Kitam., Dicliptera chinensis (L.) Nees, Desmodium heterocarpum (L.) DC., Setaria viridis (L.) Beauv., Setaria glauca (L.) Beauv., Calystegia japonica Choisy, Sericocalyx chinensis (Nees) Brem., Cynoglossum amabile Stapf et Drumm., Phyllanthus cochinchinensis Spr., Disporum calcaratum D. Don., Craibiodendron stellatum (Pier.) w. w. Smith, Vaccinium laetum Diels, P1429, Parnassia delavayi Franch., Crawfurdia fasciculate wall., Crawfurdia chinensis Migo, Gymnocladus chinensis Baill., Senecio oldhamianus Maxim., Lepus tolai Pallas, Lepus mandschuricus Radde, Lepus oiostolus Hodgson, Lepus sinensis Gray, Oryctolagus cuniculus domesticus (Gmelin), Ainsliaea glabra Hemsl., Syneilesis aconitifolia Maxim., Tanacetum sibiricum L., Pecteilis susannae (L.) Raf., Alcedo atthis bengalensis Gmelin, Chara fragilis Desv, Derris trifoliata Lour., Ichthyoxenus tchangi Yu, Mentha rotundifolia (L.) Huds., Swertia davidi Franch., Dichrocephala benthamii C. B. Clarke, Dichrocephala chrysanthemifolia (B1.) DC., Houttuynia cordata Thunb., Lepidogrammitis drymoglossoides (Bak.) Ching, Cyrtomium fortunei J. Sm., Ajuga lupulina Maxim. , Magnolia coco (Lour.) DC., Lespedeza cuneata (Dum. Cours.) G. Don, Hypserpa nitida Miers, Selaginella involvens (Sw.) Spring, P1447, Rhododendron molle (Bl.) G. Don, Rhododendron hunnewellianum , R. japonicum Suringer, Alstonia mairei Levl.,Smithsonite , Fugu ocellatus (Osbeck), Fugu vermicularis (Temminck & schlegel), Fugu obscurus (Abe), Pseudogyrinocheilus procheilus (Sauvage et Dabry), Leptochloa chinensis (L.) Nees, Aleurites fordii Hemsl., Aleurites montana, P1455, Symplocos laurina (Retz.) wall., Oxytropis oxyphylla DC., Leycesteria virescens Lindl., C. pumilum Rolfe, P1474, Hemiboea subcapitat c. B. Clarke, Dalbergia odorifera T. Chen, Acronychia pedunculata (L.) Miq., Pulicaria insignis Drumm. , Cirsium lineare (Thunb.) Sch.-Bip. var. pallidum (Kitam.) Ling, Cirsium lineare Sch.-Bip., Coreopsis lanceolata L., Asarumheterotrppoides F. Schm. var. mandshuricum (Maxim.) Kitag., Asarum sieboldii Miq., Asarum forbesii Maxim., A. maximum Hemsl., A. geophilum Hemsl., A. caudigerum Hance, A. himalaicum Hook. f. et Thoms., A. caulescens Maxim., A. longiflorum C. Y. Cheng et C. S. Yang, A. insigne Diels, A. longipedunculatum O. C. Schmidt, A. sieboldii var. seoulensis Nakai, A. sieboldii var. cineoliferum, Asarum europaeum, Ceratophyllum demersum L., Allium schoenoprasum L., Euphorbia esula L. var. cyparissoides Boiss., Eucalyptustereticornis Smith, Murdannia simplex (Vahl) Brenan,Teucrium pernyi Franch., Justicia leptostachya Hemsl., Lespedeza chinensis G. Don, Hypecoum leptocarpum Hook. f. et Thoms., Dryopteris crassirhizoma Nakai, Lunathyrium acrostichoides (Sw.) Ching, Matteucciastruthiopteris (L.) Todaro, Osmunda japonica Thunb., Blechnum orientale L., Brainia insignis (Hook.)J. Sm., woodwardia japonica (L. f.) Sm., Dryopterispeninsulae Kitag., woodwardia unigemmata (Mak.) Nakai, Polystichum squarrosum Fe, Dryopteris filixmas (L.) Schott, Cyrtomium fortunei J. Smith, Hypericum perforatum L., Mactra sulcataria Deshayes, Corallium japonicum Kishinouye, Spatholirion longifolium (Gagn.) Dunn, Eretmochelys imbricata (L.), Citrus aurantium L. var. amara Engl., Pteria margaritifera (L.), Pteria martensii (Dunker), Callicarapa bodinieri Lvl., Ardisia maculosa Mez, Phyllanthus urinaria L., Thalictrum trichopus Franch., Sorbaria arborea Schneid., Sorbaria sorbifolia (L.) A. Br., Lysimachia clethroides Duby, Cyanotis arachnoidea C. B. Clarke, Cicuta virosa L., Millettia lasiopetala (Hayata) Merr., Botrychium virginianum (L.) Sw., Rhaphiolepis indica (L.) Lindl., Lespedeza dunnii Schindl., Citrus chachiensis Hort., Citrus suavissima Tataiwanensis wall. var. glandulosissima Airy-Shaw, Physochlaina physaloides (L.) G. Don, Catalpa fargesii Bur., Misgurnus anguillicaudatus (Cantor), Hemistepta carthamoides (Buch.-Ham.) O. Ktze., Misgurnus anguillicaudatus (Cantor), Artocarpus heterophyllus Lam., Herpetospermum caudigerum wall., Lycopus lucidus Turcz., Lycopus lucidus Turcz. var. hirtus Reg., L. lucidus Turcz. var. taiwanensis Hayata, L. parviflorus Maxim., L. maackianus (Maxim.) Mak., L. coreanus Levl., L. europaeus L., Alisma plantago-aquatica L. var. orientale Samuels, Euphorbia helioscopia L., Lysidice rhodostegia Hance, Lysimachia candida Lindl., Ervatamia hainaensis Tsiang, Aster turbinatus S. Moore, Salvia cavaleriei Levl. var. simplicifolia Stib., Lamium amplexicaule L., Angiopteris officinalis Ching, Lindernia anagallis (Burm. f.) Pennell, Trichosanthes himalensis C. B. Clarke, Azurite, Alternanthera philoxeroides (Mart.) Griseb., Maesa perlarius (Lour.) Merr., Spiraea salicifolia L., Crepishookeriana C. B. Clarke, Selaginella tamariscina (Beauv.) Spring, Selaginella pulvinata (Hook. et Grev.) Maxim., Corallodiscus kinginus (Craib) B. L. Burtt, Epilobium himalayense Hausskn., Cymbidium ensifolium (L.) Sw., Cymbidium naka, Citrus suhoiensis Tanaka, C. poonensis Tanaka, C. tankan Hayata, Lithocarpus glaber (Thunb.) Nakai, Kopsia officinalis Tsiang et P. T. Li, Cudrania tricuspidata (Carr. ) Bur., Abrus precatorius L., Citrus grandis (L.) Osbeck, Citrus grandis (L.) Osbeck var. wentanyu Hort., C. grandis (L.) Osbeck var. shatinyu Hort., C. grandis (L.)Oseck var. Pinshanyu Hort., C. grandis (L.) Osbeck var. szechipow Hort., C. grandis (L.) Osbeck var. tahungpow Hort., Viscum orientale willd., Citrus aurantium L., Citrus sinensis (L.) Osbeck, C. medica L.anche caerulescens Steph., Orobanche pycnostachya Hance, Orobanche asiatica Kirschl., O. rapum-genistae Thuill., Tephrosia purp Though it is not restricted especially, Traditional Chinese medicine or/and its extracts, granulated powdered medicines manufactured from natural plants, animal parts, or mineral substances, for instance, the following can be enumerated (the traditional medicine names are represented in their Japanese pronunciation); Tsumuratou, Tsumuratou kasenkyushini, Ojji-tou, Ancyuusan, Jyuumihandokusan, Hachimijiogan, Daisaikotou, Syousaikotou, Saikokeishitou, Saikokeishikankyoutou, Saikokaryukotsuboreitou, Hangesyashintou, Ourengeddokutou, Hangekoubokutou, Goreisan, Keishikajyutsufutou, Syouseiryuutou, Boujiougitou, Syouhangekabukuryoutou, Syoufuusan, Toukisyakuyakusan, Kamisyouyousan, Keishibukuryougan, Keishikaryukotsuboreitou, Maoutou, Ebbikajyutsutou, Makumontou, shinbutou, Gosyuyutou, Ninjintou, Daioubotanpitou, Byakkoukaninjintou, Shigyakusan, Mobougitou, Hangebyakujyutsutenmatou, Toukishigyakukagosyuyusyoukyoutou, Ryoukeijyutsukantou, Cyoreitou, Hocyuuekitou, Rikkushitou, Keishitou, Shichimotsukoukatou, Koutousan, Jyuzendaihotou, Keigairengyoutou, Juncyoutou, Yokuinintou, Sokeikakketsutou, Yokkansan, Mashinkansekitou, Gorinsan, Onseiin, Seijyouboufuutou, Chitousouichihou, Keishikasyakuyakutou, Toukakusyoukitou, Boufuutsuusyousan, Gosekisan, Syakukanzoutou. Kihitou, Jinsouin, Nyojinsan, syakuyakukanzoutou, Bukuryousan, Shimotsutou, Kanmakutaisoutou, Saikantou, Cyouisyoukitou, Shikushitou, Ryutansyakantou, Toukikyouaitou, Mashinsekikantou, Heiisan, Saikoseikantou, Nichintou, Keishininjintou, Yokkansankachipihange, Daioukanzoutou, shinpitou, Toukiinshi, Rokumigan, Nijyutsutou, Chidabokuichihou, Seihaitou, Ondantou, Jiinchihoutou, Jionkoukatou, Gokoutou, Saipokutou, Daiboufuutou, Ougikencyuutou, Syoukencyuutou, Daikencyuutou, Syoumatou, Toukitou, Sansounintou, shiniseihaitou, Tsuutousan, Onkeitou, Gosyajinkigan, Ninjinyoueitou, Syousaikotoukakikkyosekkou, Rikkousan, Seishinrenshiin, Cyoreitougoushimotsutou, Sanousyashintou, Saireitou, Ireitou, Bukuryouingouhangekoubokutou, Goreisan, Ryoukyoujyutsukantou, Ryoukankyoumishingenintou, Ourentou, Sanmotsuougontou, Hainousan, Hainoutou, Toukikencyuutou, Sencyacyousan, Keishibukuryougankamashiningan, mashiningah, Maoubushisaishintou, Keihitou, Daisyoukitou, Keishikadaioutou, Inchinkoutou, Seisyoekitou, Kamikihitou, Kikkyotou, Shiyunkou, Syouyakukoujin, Shouyakushuuzibushi, Meta light, Astattocrem, Astat liquid, Astat ointment, Shinfaz, and Toconshiropp, Heiisanryou, Atsushikiyoshiin, Goreisanryou, Kobushiseihaitou, Boukiougitou, saikokaryukotsuboreitou, shinpitou, Boufuutsuseisanryou, Ourentou, Saikokeishikankyoutou, Sokeikakketsutou, Saikokeishitou, Daisaikotou, Mashinkansekitou, Kakkontou, Saikoseikantou, Koutousanryou, Mashinkantou, Kakontoukasenshini, Sanousyashintou, Cyoreitou, Mokuboukitou, Toukishigyakukagosyuyusyoukyoutou, Yokukansankachinpihange, Toukisyakuyakusanryou, Rikkunshitou, Keishikaryuukotsuboreitou, Jyuumihandokutou, Hangekoupukutou, Ryuutansyakantou, Oncyushitsuutou, Onseiin, Ourentou, Sofuuteitsuutou, Yokkansankachinpihangetou, Junketsuonhotou, Ryuudansyakantou, taiyousoubitou, Shiyakuonketsutou, Kaikinshitsuutou, Oncyuushitsuutou, keishitou, Syousyouyoujyou, Hyoushitsuseicyoujyou, Onseiin, Keireianjunjyou, Syouseiryuutou, Kouhiseikenjyou, Ourentou, Gomirisuijyou, Hocyuukaikijyou, Seinetsusyahijyou, Ourengeddokutou, Ryuukajunseijyou, Soukoujunkijyou, Kougyakusuikijyou, Tenchitsuucyoujyou, Hanrikaiyoujyou, Shimosyoutsuusuijyou,. Kaihyousoukinjyou, Sansounintou, Futokuanjunjyou, Kakkontoukasenshini, Shiyakuonketsutou, Shimotsutou, Makumontou, Hangekoupokutou, Keigairengyoutou, Hyoukaireiyoujyou, Cyuusyoukenwajyou, Hangesyashintou, Ougegankadaiou, Ourengedokugan, Kankyouninjinhangegan, Kuketsugan, Keishibukuryougan, Keiryoukadaiougan, Sanougan, Daikangan, Toukakusyoukigan, Mashiningan, Ricyuugan, Rokumigan, Hachimigan, Ancyuusan, Kamisyouyousan, Syoufuusan, tsuudousan, Toukisyakuyakusan, Hainosan, Heiisan, Ninjinyoueitou, Daioukanzoutou, Kamikihitou, Toukisyakuyakusan, Hocyuuekitou, Sairyoutou, Saikokeishitou, Goryousan, Keishibukuryougan, keishikajyutsufutou, Kamisyouyousan, Kakkontou, Ourengedokutou, etc.

All the drugs authorized by the Japan Ministry of Health and Welfare can be enumerated.

### "Compositions of the active ingredients of the agent for hair Growth"

The processed semi-mature soybean, the processed semi-mature soybean extracts, the processed Polygoni Multiflori Radix, the processed Polygoni Multiflori Radix extracts, the processed Cynanchum bungei Decne, the processed Cynanchum bungei Decne extracts and the Longan seed have the hair growth effects respectively.

However, this invention obtained synergistic effects of hair growth by blending the processed semi-mature soybean and/or its extracts and at least one substance selected from the group consisting of the processed Polygoni Multiflori Radix, its extracts, the processed Cynanchum bungei Decne and its extracts.

In this invention, notable effects of hair growth can be obtained with the addition of only small amounts of each of the processed Polygoni Multiflori Radix, the processed Cynanchum bungei Decne and Longan seed to the processed semi-mature soybean. The ratio of the mixture of the processed semi-mature soybean with the processed Polygoni Multiflori Radix, the processed Cynanchum bungei Decne, and Longan seed is not especially restricted. It is possible to adjust the amounts appropriately according to how the ingredients are combined as well as the mode of the agent for hair growth, etc. The following ranges are one mode of this invention and although they are desirable, one is not restricted within them especially.

The agent for hair growth in this invention usually contains a processed semi-mature soybean and/or a processed semi-mature soybean extracts, along with at least one substance selected from the group consisting of a processed Polygoni Multiflori Radix, processed Polygoni Multiflori Radix extract, processed Cynanchum bungei Decne or processed Cynanchum bungei Decne extract as active ingredients.

In this mode of the agent for hair growth, it is preferable to obtain enough of a synergetic effects in hair growth by mixing the ingredients under the following conditions; blend "a" parts by weight of the processed semi-mature soybean and "b" parts by weight of the processed semi-mature soybean extracts (wherein "a" and "b" are numbers that are not less than 0, and fill a relation of a+b=100) with
"c" parts by weight of the processed Polygoni Multiflori Radix , "d" parts by weight of the processed Polygoni Multiflori Radix extracts, "e" parts by weight of the processed Cynanchum bungei Decne and "f" parts by weight of the processed Cynanchum bungei Decne extracts (wherein "c", "d", "e", and "f" are numbers that are not less than 0, and fill a relation of 3≤c+d+e+f≤100, preferably 20≤c+d+e+f≤60, for example, c+d+e+f=40).

Moreover, a more desirable mode of the agent for hair growth of the invention is achieved by containing Longan seed and/or Longan seed extracts as active ingredients into the above-mentioned ingredients.

In this mode of the agent for hair growth, it is preferable to obtain enough of a synergetic effects in hair growth (especially improving the color and luster of hair) by mixing the ingredients at the rates under the following conditions;
blend "a" parts by weight of the processed semi-mature soybean and "b" parts by weight of the processed semi-mature soybean extracts (wherein "a" and "b" are numbers that are not less than 0, and fill a relation of a+b=100) and
"c" parts by weight of the processed Polygoni Multiflori Radix , "d" parts by weight of the processed Polygoni Multiflori Radix extracts, "e" parts by weight of the processed Cynanchum bungei Decne and "f" parts by weight of the processed Cynanchum bungei Decne extracts (wherein "c", "d", "e", and "f" are numbers that are not less than 0, and fill a relation of 3≤c+d+e+f≤100, preferably 20≤c+d+e+f≤60, for example, c+d+e+f=40) with
"g" parts by weight of the Longan seed and "h" parts by weight of the Longan seed extracts (wherein "g" and "f" are numbers that are not less than 0, and fill a relation of 2≤g+h≤30, preferably 4≤g+h≤12, for example, g+h=8).

### "The mode of the agent for hair geowth"

As far as efficacy is concerned, in the agent for hair growth of this invention, oral administration is suitable. For instance, the powder or liquid (extracts) form of the agent can be taken by adding an additive such as sweeteners if necessary. As an example, various forms of oral administration of the agent for hair growth are enumerated

These may include the liquid type such as syrup, an ampuled liquid (beverages), and then tablet, capsule, powder, fine granule and granulated powder form. In the case of oral administration, a variety of well-known substances such as bonding agents, forming agents, explosive puff agents, lubricants, brightening agents, sweeteners, and zests, etc. can be used for the agent for hair growth of this invention. Here, the tablets can be covered with shieracc or sugar. Moreover, the capsule preparations may contain a liquid catalytic supporting substance such as oils and lipids amongst the materials mentioned above. The syrup and ampuled liquid preparations (beverages) may contain sweeteners, preservatives, and coloring and flavoring substances.

Additionally, it is possible to take the agent for hair growth of this invention together with the following foods; Western-style cakes such as cookies; Rice crackers, sweet jelly made from bean jam, Japanese-style confections such as Taiyaki, Chinese cakes such as Geppei, boiled dumplings etc.; or Chinese-style raw food substances; Foods in daily life such as breads, noodles, and rice balls etc.; various kind of raw materials of food; Eating and drinking substances such as candies and juices. One is not restricted by these additional methods. Various methods can be used when combining with conventional foods and beverages. In such cases, the amount of the agent for hair growth of this invention used can be appropriately adjusted according to an individual's eating and drinking habits.

When the agent for hair growth of this invention is used orally, the frequency and the amount of intake can be properly adjusted to the relative conditions, it is not restricted especially.

For instance, intake of the agent twice a day at the following times; when hungry in the early morning and before going to sleep at night. Also, the agent can be taken orally at a frequency ranging from once to several times a day. To achieve the hair growth effects, ingesting 0.08g to 0.5g/kg body weight/day of the processed semi-mature soybean is sufficient. The Japanese Ministry of Health, Labour and Welfare recommends that the amount of the soybean intake is 76g to 100g per day. "National health care movement of the 21st century (health Japan 21)" (Ministry of Health and Welfare: 2000). The agent for hair growth of this invention can achieve noticeable results of hair growth even if it is ingested for only a short period of time, such as 6-12 weeks.

In addition, the agent for hair growth of this invention can be used as an external hair tonic. In this way, it is possible to apply it with various well-known hair treatment methods, such as hair tonics, shampoos, rinses, hair conditioners, hair treatments, and Hearorshorn, hair creams, hair oils, hair gels, ointments, powders, and granulated powders, etc.

In hair tonic such externally, aside from the active ingredients of this invention, materials such as drugs, quasi-drugs, skin cosmetic, the cosmetic raw materials and various elements mixed with conventional hair tonics (for example, materials for moisture preservation, surface-active agents, emulsifying agents, powders, coloring matters, solubilizers, cleaning agents, ultraviolet ray absorbents, thickening agents, medicines, spices, pH adjustment materials, resins, anti-bacterials, viral and mold materials, alcohols, esters, hydrocarbons, rows, and oils, lipids, fatty acids, etc.) can be properly mixed. Moreover, other compounds and other plant extracts which have hair growth effects may be used together within a certain range so as not to impair the effects of this invention.

It is desirable to apply the agent of this invention externally from once to several times per day directly onto the affected area.

### EXAMPLES

The inventor explains this invention more concretely in the following examples, but is not restricted by them.

### "making of the processed semi-mature soybean"

After a frying-pan was preheated in a gas stove, one kilogram of dry yellow soybeans was put into the frying-pan, which were stirred with medium heat for three minutes; after which the semi-mature soybean was obtained.

In the processed semi-mature soybean, the typical taste of the raw soybeans was removed without pulverizing the soybeans by excessive heating.

### "making of the processed Polygoni Multiflori Radix"

Material: 1.0 kilogram of black soybean. 2.5 liters of the Huangj iu (brandname: "FuzhouLaojiu", Fuzhou Zhaojiu Chang, Fuzhou China) . 10 kilograms of raw Polygoni Multiflori Radix. (cultivated in Shandong province, China, and obtained from Pinghe prefectural hospital, China.)

Procedure 1: 1.0 kilogram of black soybeans is added to 5 liters of water and brought to a boil by using a low flame for 3 hours to obtain 1.0 liter of hot water extract. 1.5 liters of hot water extract is obtained with the second extraction.

Procedure 2: Mix 2.5 liters of the hot water extract obtained in procedure 1 and 2.5 liters of Huangjiu. Then, add this mixed solution into 10 kilogram of raw Polygoni Multiflori Radix, heat for about 10 hours by using hot water, soak all of the black soybean extracts and Huangjiu into the Polygoni Multiflori Radix.

Procedure 3: Dry the Polygoni Multiflori Radix obtained in procedure 2 by using a natural drying method.

### "making of the processed Cynanchum bungei Decne"

Material: 1.0 kilogram of black soybean, 2.5 liters of Huangjiu (brandname: "Fuzhou Laojiu", Fuzhou Zhaojiu Chang, Fuzhou China). 10 kilograms of raw Cynanchum bungei Decne. (cultivated in Shandong province, China, and obtained from Pinghe prefectural hospital, China.)
Procedure 1: Add the 1.0 kilogram of black soybeans to 5 liters of water and boil by using a low flame for 3 hours, obtaining 1.0 liter of hot water extract. 1.5 liters of hot water extract is obtained with second extraction.
Procedure 2: Mix 2.5 liters of the hot water extract obtained in procedure 1 and 2.5 liters of Huangjiu. Then, add this mixed solution into 10 kilograms of raw Cynanchum bungei Decne, heat for about 10 hours by using hot water, soak all of the black soybean extracts and Huangjiu into the Cynanchum bungei Decne.
Procedure 3: Dry the Cynanchum bungei Decne obtained in procedure 2 by using a natural drying method.

### "making of the processed Longan seed"

Material: 1.0 kilogram of black soybeans, 2.5 liters of the Huangjiu (brandname: "Fuzhou Laojiu", Fuzhou Zhaojiu Chang, Fuzhou China). 10 kilograms of raw Longan seeds (cultivated in Shandong province, China, and obtained from Pinghe prefectural hospital, China.)
Procedure 1: Add 1.0 kilogram of the black soybeans to 5 liters of water and boil by using a low flame for 3 hours, obtaining 1.0 liter of hot water extract. 1.5 liters of hot water extract J is obtained with the second extraction.
Procedure 2: Mix 2.5 liters of the hot water extract obtained in procedure 1 and 2.5 liters of Huangjiu. Then, add 10 kilograms of Longan seed to this mixed solution, heat for about 10 hours by using hot water, soaking the Longan seed in all of the black soybean extract and Huangjiu.
Procedure 3: Dry the Longan seed obtained in procedure 2 by using a natural drying method.

### Subject

In this invention, the subjects were 70 healthy individuals (59 males and 11 females) ranging in age from 29 to 53 years old (the average age is 40.1+/-5.8 years old.) The merits and risks of participating in this research experiment were explained by myself to all the subjects and subsequently received their approval. All subjects involved suffer from alopecia and/or thinning hair.

### The groups and the hair restoration ingredients

The subjects were divided into 14 groups with each group consisting of 5 individuals and received the following respective hair restoration powder substances for oral consumption.
1, The raw soybean group: the subjects ingested raw yellow soybean powder (100%).
2, The processed semi-mature soybean group: the subjects ingested processed semi-mature soybean powder (100%).
3, The mature yellow soybean group: the subjects ingested mature yellow soybean powder (100%). This was obtained by steaming the beans for two or more hours until having completely matured.
4, The boiled black soybean group: the subjects ingested boiled black soybean powder (100%).
5, The raw Polygoni Multiflori Radix group: the subjects ingested dry raw Polygoni Multiflori Radix powder (100%).
6, The processed Polygoni Multiflori Radix group: the subjects ingested processed Polygoni Multiflori Radix powder (100%).
7, The raw Cynanchumbungei Decne group: the subjects ingested dry raw Cynanchum bungei Decne powder (100%).
8, The processed Cynanchum bungei Decne group: the subjects ingested processed Cynanchum bungei Decne powder (100%).
9, The Huangjiu group: the subjects ingested Huangjiu.
10, The Longan seed group: the subjects ingested dry Longan seed powder (100%).
11, The processed Longan seed group: the subjects ingested processed Longan seed powder (100%).
12, The "compound ingredients 1" group: the subjects ingested the "compound ingredients 1" powder which consisted of the processed semi-mature soybean and the processed Polygoni Multiflori Radix at a ratio of 2.5 : 1.0 (w : w) respectively.
13, The "compound ingredients 2" group: the subjects ingested the "compound ingredients 2" powder which consisted of powders of the processed semi-mature soybean and the processed Cynanchum bungei Decne with a ratio of 2.5 : 1.0 (w : w) respectively.
14, The "compound ingredients 3" group: the subjects ingested the "compound ingredients 3" which consisted of powders of processed semi-mature soybeans, processed Polygoni Multiflori Radix and Longan seeds with a ratio of 12.5 : 5 : 1(w : w : w) respectively.

### Dosage and frequency

The raw yellow soybean powder, the processed semi-mature soybean powder, the mature yellow soybean powder and the boiled black soybean powder were taken twice a day - once before breakfast and again once before retiring at night-time, with a dosage of 8g each time, ingesting a total of 16g per day.

The raw Polygoni Multiflori Radix, the processed Polygoni Multiflori Radix powder, the raw Cynanchum bungei Decne powder, the processed Cynanchum bungei Decne powder, the Longan seed powder and the processed Longan seed powder were taken twice per day-once before breakfast and again before retiring at night-time, with a dosage of 4g each time, ingesting 8g in total per day.

The Huangjiu was taken once per day before retiring at night-time with a dosage of 200 ml each time.

The "compound ingredients 1", the "compound ingredients 2", the "compound ingredients 3" were taken twice a day - once before breakfast and once before retiring at night-time, with a dosage of 12g each time, ingesting 24g in total per day.

### Evaluation of state of hair

Regarding the method of evaluating the extent of alopecia and area of thinning hair, a well-known method was adopted which includes dividing the area of thinning hair into six stages representing the following conditions:
A condition of normal health is termed "stage 0"; no hair growing in the area of the forehead and the rest of the head as "stage 5"; and stages between 0 and 5 are divided into stages 1, 2, 3, and 4. When difficult to differentiate clearly, it was acceptable to use decimal values such as "0.5".

Moreover, the degree of pain experienced when hair was pulled was divided into three stages as an index to evaluate the effects of hair growth. That is, "degree 1" indicated mild-, "degree 2" moderate-, and "degree 3" strong pain sensation.

This data was calculated by using the Student's "t" test with Microsoft Excel 2003 and was significant when p equated to less than 5%.

Moreover, when a subject gave consent, pictures were taken using a camera (MZ50) under same lighting conditions.

Furthermore, some subject's hair was cut at a length of 20 mm. (However, out of respect for the dignity of the individual subjects, the hair of those individuals who wished for their hair not to be cut was compared to their usual hairstyle to monitor any changes in hair growth.)

### Time points of evaluation

Data was collected and evaluated at the following times: before the test, at the end of the 6th week and again at the end of the 12th week. However, in the following description, the data gathered before the test and that gathered at the end of the 12th week was used.

### Results

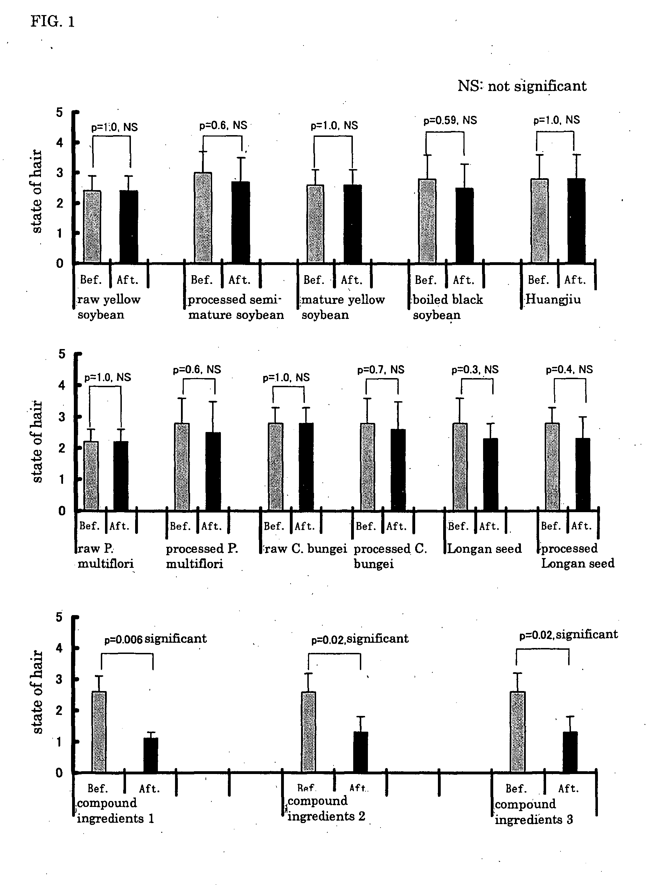

Figure 1 shows the results for the alopecia and areas of thinning hair. In the cases when the raw yellow soybeans, the mature yellow soybeans, the Huangjiu, the raw Polygoni Multiflori Radix and the raw Cynanchum bungei Decne were taken respectively, no improvement of the alopecia and/or areas of thinning hair was observed. These results show that there was no hair restorative effect when these materials were taken respectively.

In addition, in those cases when the processed semi-mature soybean, the boiled black soybean, the processed Polygoni Multiflori Radix, the processed Cynanchum bungei Decne, Longan seeds or the processed Longan seeds were taken respectively, little improvement in the alopecia and/or areas of thinning hair was found. However, there were no significant changes in the statistics (each p>0.05, Figure 1). On the other hand, these results showed that these ingredients do have some effect of hair restoration when taken respectively.

Also, in those cases when the "compound ingredients 1", the "compound ingredients 2" and the "compound ingredients 3" were taken respectively, significant improvements in the alopecia and/or areas of thinning hair were found. This could not be verified when taking the ingredients independently as was the case with groups 2, 4, 6, 8, 9 and 10.

Demonstrating the synergistic effects of ingredients used in combination to promote hair restoration, adding to significant changes in the statistics (each p<0.05, Figure 1).

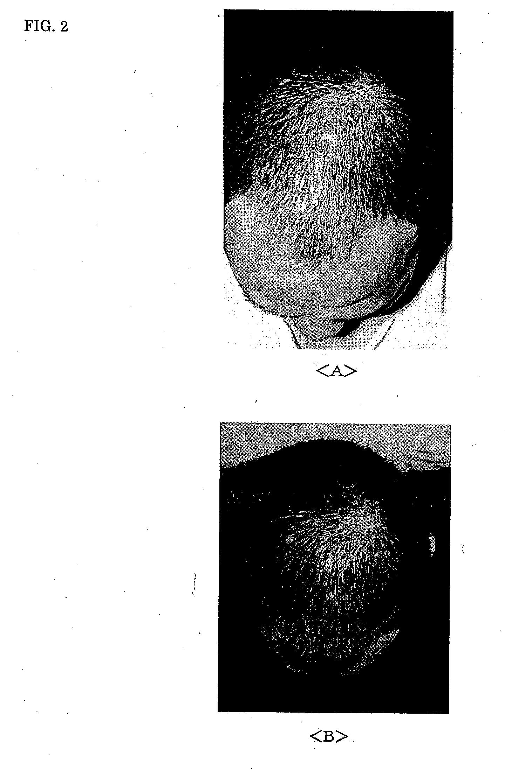

Figure 2 is photographic data of a subject who took "compound ingredients 1", proving that this set of ingredients 1 had an excellent effect on hair growth (the Hatsumou).

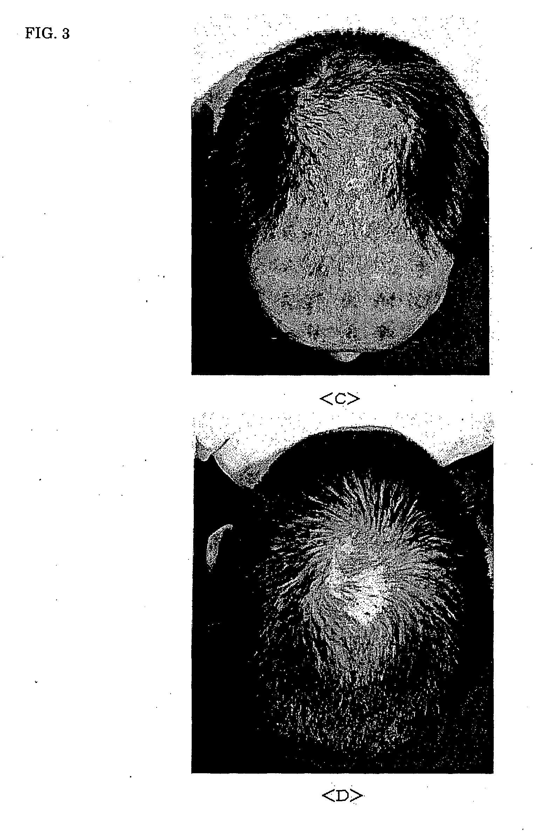

Figure 3 is photographic data of a subject who ingested "compound ingredients 2", proving that this particular combination also had an excellent effect on hair growth (the Hatsumou).

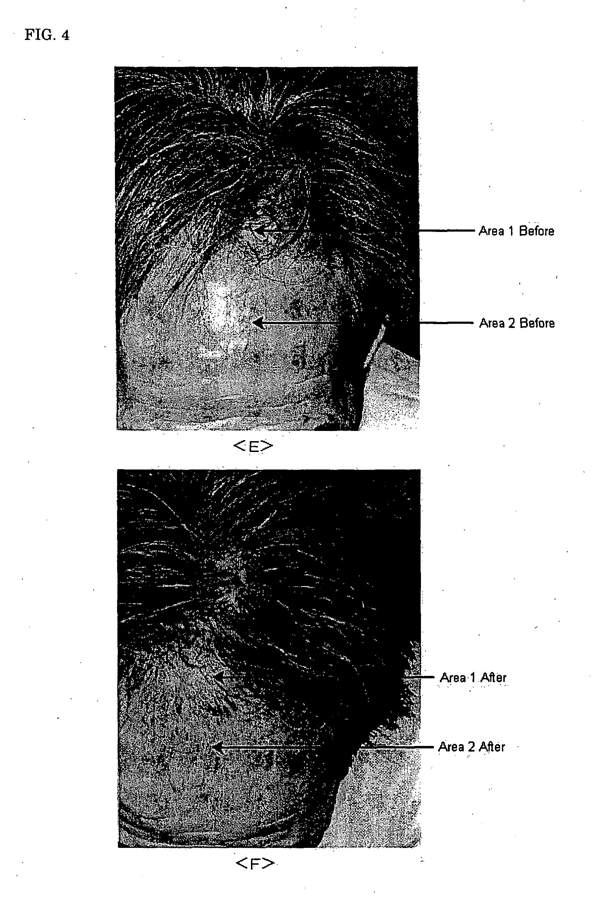

Figure 4 is photographic data of a subject who took "compound ingredients 3". This individual participated in this test for the sake of improving his thinning and greying hair. After taking "compound ingredients 3" for 12 weeks, his thinning hair improved, in density and more than half of his gray hair turned black. These effects were based on mixing Longan seeds with the "compound ingredients 1" or "compound ingredients 2".

Moreover, Area 1 and Area 2 had already experienced hair loss about 25 years ago. The Hatsumou could be confirmed even in these two areas thereby proving that "compound ingredients 3" had an excellent effect on hair growth.

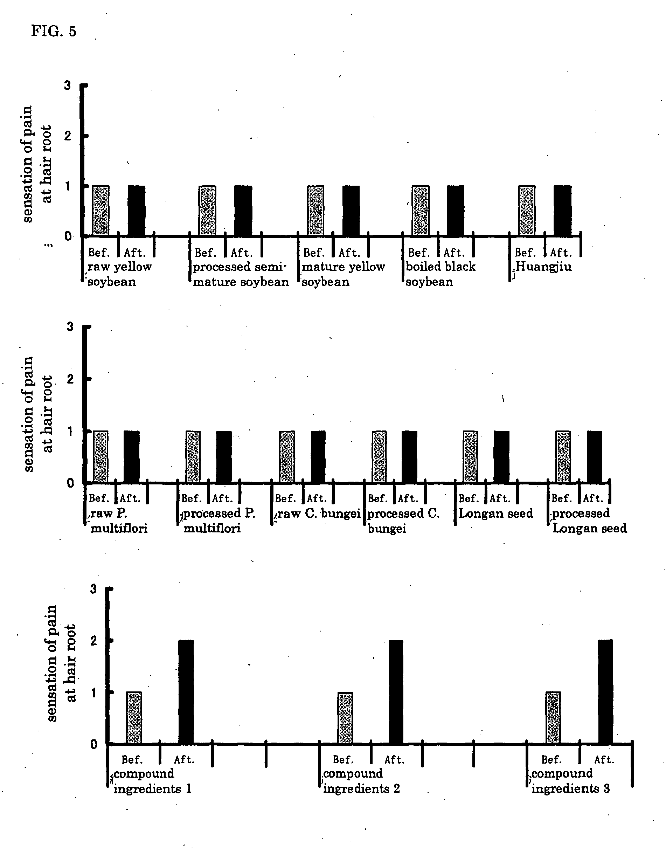

Figure 5 shows the changes in pain sensation at the hair root. In those cases where the hair restoration ingredients were taken independently, no changes in pain at the hair root were found when the hair was pulled. Additionally, in those cases where "compound ingredients 1", "compound ingredients 2" or "compound ingredients 3" were taken respectively, the pain in the hair root became more perceptible.

In alopecia and the thinning of hair, it is well-known to be accompanied by shrinkage of the hair root. Current hair tonics aim to mainly promote the circulation of blood and replenish nourishment however no reports have been found documenting pain in the hair root.

This inventor found that a person with alopecia or thinning of hair either does not feel pain/or experiences only slight pain when his/her hair is pulled. The inventor thought that this phenomenon can be used as an evaluation index of hair growth. By taking the agent for hair growth of this invention, the pain at the hair root became perceptible. The reasons for this are thought to be that the agent for hair growth of this invention either improved the health of the hair and its root, or that the nervous function of the hair root was improved.

Additionally, though it does not present in this description as data, all the subjects of groups 12, 13 and 14 experienced notable improvement in the sheen of hair.

Moreover, in this description, the results at the time of the 12th week were used. It is thought that further effects of hair restoration will appear by continuing this test.

Furthermore, at no time during which the compounds were taken were any side effects observed. Come to think of it, soybeans (yellow soybeans and black soybeans) have a long history in their use as food - such as Natto and bean curd in Japan - thereby clearly proving their safety. Huangjiu also has a history of 3000 years or more as a kind of beverage in China, and in Japan a history of over 200 years. It has been proven that drinking Huangj iu in appropriate quantities positively influences one's health. The processed Polygoni Multiflori Radix and the processed Cynanchum bungei Decne have 1000 years or more history and were used as "Buyiyao" (meaning herbal tonics, a term of traditional Chinese Medicine), without any documented side effects. In recent years, the effect of processed Polygoni Multiflori Radix in decreasing serum cholesterol was found, and it is also widely used for the treatment and prevention of geriatric diseases. The Ministry of Health and Welfare in Japan has also aknowledged the medicinal of the plant and has included it in the "Japanese Pharmacopeia", authorizing it as an oriental medicine in Japan.

Data is not shown in this description, however in regards to some of the subjects who ingested the "compound ingredients 1", the "compound ingredients 2 "or the "compound ingredients 3", their blood-, hepatic- and kidney function were examined. The results concluded that there were no side effects at all. In one group of the subjects, the values in their data became more normal. Therefore, it has been proven that the compound ingredients are safe for ingestion.

Based on the above results, it became clear that the "compound ingredients 1", the "compound ingredients 2" and the "compound ingredients 3" have excellent effects on hair growth, can notably stimulate hair growth in a short period of time, and that each compound is safe for consumption. In addition, it can be said that the compounds of this invention have the added value that is not inherent in conventional hair tonics, namely that it can be processed into the form of delicious foods whilst having excellent hair growth effects.

### INDUSTRIAL APPLICABILITY

The agent for hair growth of this invention can be used as a medicine by an untouched mode as well as a food and beverage additive. Furthermore, the agent can be preserved under room temperature for a moderate to long period of time (from half a year to 2 years) . The raw materials can be procured from regular markets, thereby ensuring its industrial applicability.

## Claims

1. An agent for hair growth **characterized by** comprising:
a processed semi-mature soybean and/or a processed semi-mature soybean extracts and
at least one substance selected from the group consisting of a processed Polygoni Multiflori Radix, processed Polygoni Multiflori Radix extracts, a processed Cynanchum bungei Decne or processed Cynanchum bungei Decne extracts as active ingredients,
wherein the processed semi-mature soybean is obtained by heating soybeans within the temperature range that removes the typical of raw soybean taste without pulverizing the soybeans by excessive heating and
the processed Polygoni Multiflori Radix or the processed Cynanchum bungei Decne are each obtained by soaking black soybean extracts and liquors into raw Polygoni Multiflori Radix or raw Cynanchum bungei Decne.

2. The agent for hair growth according to claim 1, which comprises
"a" parts by weight of the processed semi-mature soybean and "b" parts by weight of the processed semi-mature soybean extracts (wherein "a" and "b" are numbers that are not less than 0, and fill a relation of a+b=100) with
"c" parts by weight of the processed Polygoni Multiflori Radix , "d" parts by weight of the processed Polygoni Multiflori Radix extracts, "e" parts by weight of the processed Cynanchum bungei Decne and "f" parts by weight of the processed Cynanchum bungei Decne extracts (wherein "c", "d", "e", and "f" are numbers that are not less than 0, and fill a relation of 3≤c+d+e+f≤100).

3. The agent for hair growth according to claim 1, which further comprises Longan seed and/or Longan seed extracts as active ingredients.

4. The agent for hair growth according to claim 3, which comprises
"a" parts by weight of the processed semi-mature soybean and "b" parts by weight of the processed semi-mature soybean extracts (wherein "a" and "b" are numbers that are not less than 0, and fill a relation of a+b=100) and
"c" parts by weight of the processed Polygoni Multiflori Radix , "d" parts by weight of the processed Polygoni Multiflori Radix extracts, "e" parts by weight of the processed Cynanchum bungei Decne and "f" parts by weight of the processed Cynanchum bungei Decne extracts (wherein "c", "d", "e", and "f" are numbers that are not less than 0, and fill a relation of 3≤c+d+e+f≤100) with
"g" parts by weight of the Longan seed and "h" parts by weight of the Longan seed extracts (wherein "g" and "f" are numbers that are not less than 0, and fill a relation of 2≤g+h≤30).

5. The agent for hair growth according to any one of claims 1 to 4, which is prepared as an oral type.
